# EUROPEAN PATENT SPECIFICATION

(11) **EP 0 777 453 B1**
(45) Date of publication and mention of the grant of the patent: **02.11.2000**
(21) Application number: 95930896.6
(22) Date of filing: 21.08.1995
(51) Int. Cl.: A61F 13/26

(54) **TAMPON APPLICATOR HAVING AN IMPROVED PLEATED TIP**
TAMPONAPPLIKATOR MIT EINER VERBESSERTEN GEFALTETEN SPITZE
APPLICATEUR DE TAMPON A BOUT PLISSE AMELIORE

(30) Priority: 22.08.1994 US 294229; 06.09.1994 US 300987
(43) Date of publication of application: 11.06.1997
(73) Proprietor: Kimberly-Clark Worldwide, Inc., Neenah, WI 54957-0349 (US)
(72) Inventor: NIELSON, Steven, James, Greenville, WI 54942 (US); KRUEGER, Allan, James, Winneconne, WI 54986 (US); RASMUSSEN, Noel, John, Oshkosh, WI 54901 (US); RENTMEESTER, Tammy, Jo, Appleton, WI 54915 (US); TEWS, Richard, Roy, Larsen, WI 54947 (US); WEYENBERG, Jeffrey, Michael, Appleton, WI 54915 (US)
(74) Representative: DIEHL GLAESER HILTL & PARTNER
(86) International application number: US9510659
(87) International publication number: WO9605794

(56) References cited:
- US-A- 3 358 354
- US-A- 3 433 225
- US-A- 3 753 437
- US-A- 4 361 150

## Description

### FIELD OF THE INVENTION

This invention relates to a tampon applicator having an improved pleated tip for facilitating insertion of a catamenial tampon into a body cavity. More specifically, this invention relates to crimping, pleating and forming an insertion tip on an end of a paper tampon applicator.

### BACKGROUND OF THE INVENTION

Catamenial tampons and other types of absorptive media are routinely inserted into body cavities, such as a woman's vagina, to absorb menstrual fluid, blood and other kinds of body fluid. One convenient way to position such absorbent tampons into a body cavity is through the use of an applicator. Comfortable and clean insertion of the absorbent tampon are keys to repeated sale of such applicators. In addition, the applicator should be capable of inserting the absorbent tampon into the body cavity using only a minimum amount of expulsion force.

Tampon applicators are available in a variety of shapes and sizes with the two piece telescopically assembled design being the most prevalent. In the two piece applicator, the tampon is housed in an outer tube and is expelled into a woman's vagina by an inner member which is telescopically mounted in the outer tube and acts as a plunger. Some tampon applicators utilize a hollow tube having an open insertion end through which the tampon is always exposed while other applicators utilize a completely closed or partially closed design. A thin film membrane can cover the insertion end of an applicator to completely enclose the forward end of a tampon while folds and pleats can be used to partially enclose the forward end of a tampon and protect it from contamination. Still other applicators, especially plastic applicators, have a plurality of flexible petals formed on the forward end of the outer tube which can flex radially outward to allow the tampon to be expelled. It will be appreciated that the diameter of the applicator, the material from which it is formed, the basic configuration of the applicator, the size and shape of the tampon positioned in the applicator, as well as the ease of opening the forward end of the applicator will all influence the force required to expel the tampon therefrom. The expulsion force should be kept reasonably low to permit proper functioning of the applicator.

US-A-3 358 354 discloses a catamenial tampon device which incorporates an outer, generally cylindrical tube having a smooth, generally conically shaped forward end and methods of making same.

While many have tried to design and manufacture tampon applicators having these improved qualities, there still remains a need for a tampon applicator which is more comfortable to use. Those applicators having an open forward end tend to expose the dry absorbent fibers of the tampon to the interior walls of a woman's vagina and this can cause irritation during insertion. Commercially available plastic applicators, using a plurality of petal tips, separated by slots, can sometimes pinch or cut the vaginal tissue to a woman during insertion and cause discomfort. Paper applicators having partially of fully closed tips tend to require an increased expulsion force to expel the tampon from the applicator and this can cause the applicator to deform or cause the tampon to be inserted incorrectly. Such insertion can cause discomfort to the user.

Now a paper tampon applicator has been invented having an improved pleated tip for facilitating comfortable insertion of an absorbent tampon into a woman's vagina while requiring a low expulsion force. In addition, an apparatus and method for crimping, pleating and forming an insertion tip on the end of a paper tampon applicator has been invented which provides a central aperture formed through the insertion tip. The central aperture allows the pleats to open with a minimum amount of expulsion force and provides a visual means for the consumer to verify that the applicator does contain an absorbent tampon.

### SUMMARY OF THE INVENTION

Briefly, this invention relates to a paper tampon applicator having an improved pleated tip for facilitating insertion of a catamenial tampon into a woman's vagina. The tampon applicator includes a first member capable of housing an absorbent tampon. The first member has a central longitudinal axis and first and second ends. An insertion tip is integrally formed on the first end of the first member and extends outwardly therefrom. The insertion tip includes a semi-spherical shaped portion and a frusto-conical shaped portion. The semi-spherical shaped portion has a small central aperture formed therethrough and the aperture has a side wall which is aligned essentially parallel to the central longitudinal axis of the first member. The frusto-conical shaped portion is situated between the semi-spherical shaped portion and the first end of the first member. The insertion tip contains a plurality of pleats capable of expanding radially outward as the tampon is expelled from the first member. The tampon applicator further includes a second member telescopically mounted in the second end of the first member. The second member is adapted to expel the tampon through the insertion tip as it is pushed into the first member.

The tampon applicator is also disclosed in combination with a catamenial tampon having a shaped nose which approximates the interior surface of the first member.

The invention also relates to an apparatus and method for crimping, pleating and forming a tip on a hollow tube. The apparatus includes a first punch having a tubular section sized to receive the tube and having a configured tip with a plurality of elongated grooves formed therein. The first punch also contains a shoulder formed at an opposite end of the tubular section which acts as a stop for the tube. The first punch and tube are mateable with a first die. The first die includes a base having a plurality of blades extending axially outward therefrom. Each blade is designed to engage with one of the grooves formed on the first punch and causes the tip of the tube to be crimped therebetween. After an end of the tube has been crimped, it is transformed into a plurality of pleats and configured into a semi-spherical configuration having a central aperture formed therethrough. This is accomplished using a second punch having a tubular section sized to receive the tube. The second punch also has a semi-spherically shaped tip with a pin extending outward from the apex thereof and a shoulder formed at an opposite end of the tubular section which acts as a stop for the tube. The second punch and tube are mateable with a second die. The second die includes a base having a semi-spherical cavity formed therein with a central opening formed at the bottom of the cavity. The cavity is sized to receive the second punch and the tube, and the opening is sized to receive only the pin. The method of engaging the punches and dies to crimp, pleat and form one end of the tube is also described.

The general object of this invention is to provide a paper tampon applicator having an improved pleated tip for facilitating insertion of a catamenial tampon into a body cavity. A more specific object of this invention is to provide an apparatus and method for crimping, pleating and forming an insertion tip on an end of a paper tampon applicator.

Another object of this invention is to provide a tampon applicator having a uniquely formed tip which prevents premature contamination yet substantially encloses the forward end of an absorbent tampon.

Still another object of this invention is to provide a tampon applicator having a pleated tip which essentially encloses the forward end of an absorbent tampon and which can be opened with a minimum amount of force.

A further object of this invention is to provide an apparatus for crimping, pleating and forming the insertion end of a paper tampon applicator into a semi-spherical configuration having a central aperture formed therethrough.

A further object of this invention is to provide a tampon applicator which is economical to manufacture and easy to use.

Still another object of this invention is to provide an apparatus which is simple to build and easy to operate.

Still another object of this invention is to provide a method for crimping, pleating and forming the insertion end of a tampon applicator at high speeds.

Still further, an object of this invention is to provide a simple and economical method of crimping, pleating and forming the insertion end of a tampon applicator.

Still further, an object of this invention is to provide a paper tampon applicator which will minimize discomfort to a woman when she inserts an absorbent tampon into her vagina.

Still further, an object of this invention is to provide a spirally wound, convolutely wound, or longitudinally seamed paper tampon applicator with an improved tip for facilitating insertion of an absorbent tampon into a woman's vagina.

Other objects and advantages of the present invention will become more apparent to those skilled in the art in view of the following description and the accompanying drawings.

### BRIEF DESCRIPTION OF THE DRAWINGS

Fig. 1 is a perspective view of a two piece, spirally wound paper tampon applicator.

Fig. 2 is a cross-sectional view of a tampon applicator shown in Fig. 1.

Fig. 3 is a left end view of the tampon applicator shown in Fig. 1 depicting eight pleats.

Fig. 4 is a cross-sectional view of the insertion tip taken along line 4--4 of Fig. 3 showing an aperture formed through the insertion tip and the aperture having a side wall aligned essentially parallel to the central longitudinal axis of the first member.

Fig. 5 is a cross-sectional view of an insertion tip integrally formed on the first member and having an aperture formed therethrough wherein the side wall of the aperture is aligned at an angle to the central longitudinal axis of the first member. Fig. 5 is not according to the invention.

Fig. 6 is an alternative end view of a tampon applicator depicting three pleats.

Fig. 7 is still another alternative end view of a tampon applicator depicting sixteen pleats.

Fig. 8 is a schematic view of a pleat taken along line 8--8 of Fig. 3 depicting the shape and thickness of a pleat.

Fig. 9 is a cross-sectional view of the insertion tip taken along line 9--9 of Fig. 3 depicting one end of the pleats extending into the frusto-conical shaped portion.

Fig. 10 is a cross-sectional view of an alternative embodiment of the insertion tip showing one end of the pleats terminating at a point where the frusto-conical shaped portion is joined to the first end of the first member.

Fig. 11 is a cross-sectional view of another embodiment of the insertion tip showing one end of the pleats terminating at a point where the semi-spherical shaped portion is joined to the frusto-conical shaped portion.

Fig. 12 is a perspective view of the tampon applicator showing the pleats in an open arrangement.

Fig. 13 is a perspective view of a paper tampon applicator having an inner tube and an outer tube.

Fig. 14 is a cross-sectional view of the tampon applicator shown in Fig. 13.

Fig. 15 is a left end view of the tampon applicator shown in Fig. 13 depicting eight pleats.

Fig. 16 is a schematic view of a pleat taken along line 16--16 of Fig. 15 depicting the shape and thickness of a pleat.

Fig. 17 is a side elevational view of the outer tube before the insertion tip is formed.

Fig. 18 is a right end view of the outer tube shown in Fig. 17.

Fig. 19 is a perspective view of a first punch.

Fig. 20 is a perspective view of a first punch having a plurality of grooves formed in the tip and showing the first punch being mateable with a first die.

Fig. 21 is a partial section view showing the first punch and outer tube mating with the first die.

Fig. 22 is a side elevational view of the outer tube showing the insertion tip after undergoing crimping.

Fig. 23 is a right end view of the outer tube shown in Fig. 22.

Fig. 24 is a perspective view of the second punch and the second die with the outer tube shown in phantom.

Fig. 25 is a partial section view showing the second punch and outer tube mating with the second die.

Fig. 26 is a side elevational view of the outer tube showing the insertion tip after undergoing crimping, pleating and forming into a semi-spherical configuration.

Fig. 27 is a right end view of the outer tube shown in Fig. 26.

Fig. 28 is a perspective view of the tampon applicator showing the pleats in an open arrangement after the tampon has been expelled by the inner tube.

### DETAILED DESCRIPTION OF THE PREFERRED EMBODIMENTS

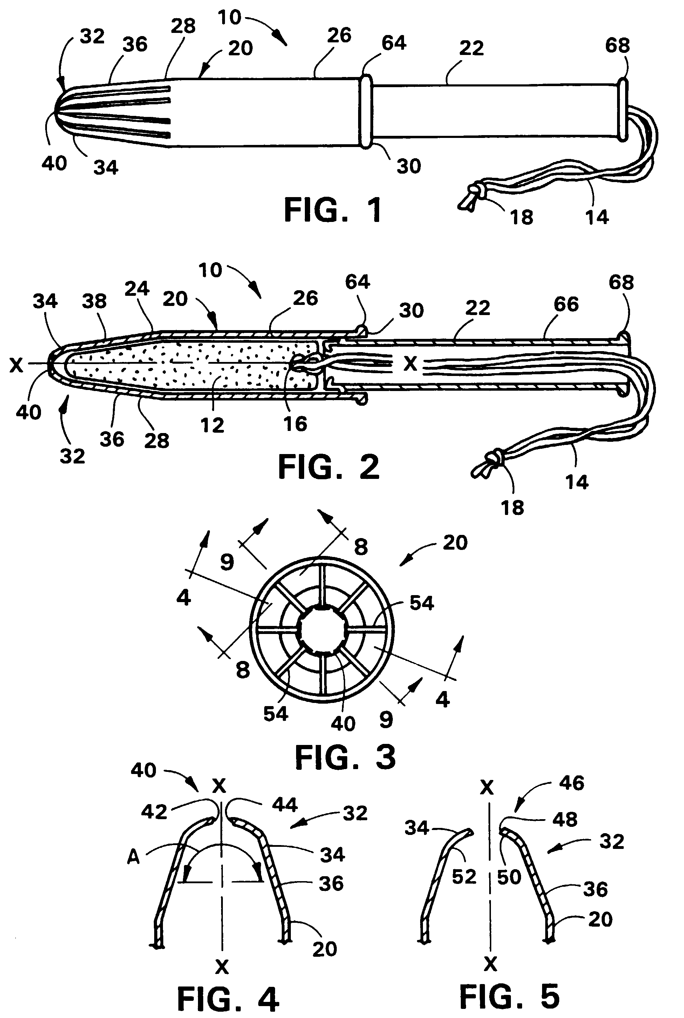

Referring to Figs. 1-3, a tampon applicator 10 is shown which is designed to house a catamenial tampon 12 and provide a comfortable means of inserting the tampon 12 into a woman's vagina. A tampon is an absorbent member primarily designed to be worn by a woman during her menstrual period to absorb menses, blood and other body fluid. The tampon 12 can be made from natural or synthetic fibers including cellulose fibers such as cotton or rayon, or artificial fibers such as polyester, polypropylene, nylon or blends thereof. Other types of fibers may also be used, such as cellulose sponge or a sponge formed from elastomeric materials. A blend of cotton and rayon fibers works well.

The tampon 12 is normally compressed into the form of a cylinder and can have a blunt, rounded or shaped forward end. The tampon 12 commonly has a withdrawal string 14 fastened to an end thereof which serves as a means for withdrawing the soiled tampon from the woman's vagina. The withdrawal string 14 can be looped through an aperture 16 formed transversely through the tampon 12. In addition, the withdrawal string 14 can have a knot 18 formed at it's free end to assure that the string 14 will not separate from the tampon 12.

The tampon applicator 10 includes a first member 20 and a second member 22. The first member 20 is preferably in the form of a spirally wound, convolutely wound or longitudinally seamed, hollow tube which is formed from paper, paperboard, cardboard or a combination thereof. The first member 20, also commonly referred to as an outer tube, is fairly rigid and has a relatively small diameter of about 10 mm to about 20 mm. The first member 20 has a wall 24 with a predetermined thickness of about .2 mm to about .6 mm. The wall 24 can be constructed from a single ply of material or be formed from two or more plies which are bonded together to form a laminate. The use of two or more plies or layers is preferred for it enables the manufacture to use certain material in the various layers which can enhance the performance of the tampon applicator 10. When two or more plies are utilized, all the plies can be spirally wound, convolutely wound, or longitudinally seamed to form an elongated cylinder. The wall 24 can be constructed using a smooth thin ply of material on the outside or exterior surface 26 which surrounds a coarser and possibly thicker ply. When the wall 24 contains at least three plies, the middle ply can be the thicker ply and the interior and exterior plies can be smooth and/or slippery to facilitate expulsion of the tampon 12 and to facilitate insertion of the first member 20 into a woman's vagina, respectively. By sandwiching a thick, coarser ply of material between two thin, smooth plies, an inexpensive first member 20 can be provided which is very functional. The wall 24 should contain one to four plies, although more plies can be utilized if desired.

The plies forming the wall 24 can be held together by an adhesive, such as glue, or by heat, pressure, ultrasonics, etc. The adhesive can be either water-soluble or water-insoluble. A water-soluble adhesive is preferred for environmental reasons in that the wall 24 will quickly break apart when it is immersed in water. Such immersion will occur should the first member 20 be disposed of by flushing it down a toilet. Exposure of the first member 20 to a municipal's waste treatment plant wherein soaking in water, interaction with chemicals and agitation all occur, will cause the wall 24 to break apart in a relatively short period of time.

The inside diameter of the first member 20 is usually less than about .75 inches (about 19 mm) and preferably less than about .625 inches (about 16 mm). Although the exterior diameter of tampons do vary, most tampons utilized by women have a external diameter of less than about .75 inches (about 19 mm). However, if one desired to use this invention to administer medication to an animal, such as a farm animal, larger size tampons 12 could be used.

It should be noted that the first member 20 can be spirally wound, convolutely wound or longitudinally seamed into a cylindrical tubular shape. Any of these methods of forming a tubular configured first member 20 is advantageous especially when the first member 20 is formed from a laminate. The reason for this is that when a laminate is circumferentially wound into a tube and a butt seam or an overlap is formed, the butt seam or the overlap can interfere with the later formation of pleats on the forward end thereof. A common problem with a rigid or stiff walled, tubular member having a relatively small diameter and a butt seam is that the seam has a tendency to come apart after formation if exposed to certain stress forces and/or high humidity. The problem with a tubular member having an overlap is that a small portion of the wall will be thicker than the remaining portion and this will cause problems when one tries to pleat one end of the tube. Accordingly, the first member 20 should be formed into a cylindrical configuration without the presence of a butt seam or an overlap. Alternatively, the overlap near the end of the tube could be cut away so that a uniform wall thickness is present.

The first member 20 is sized and configured to house the absorbent tampon 12. As stated above, the first member 20 should have a substantially smooth exterior surface 26 which will facilitate insertion of the first member 20 into a woman's vagina. When the exterior surface 26 is smooth and/or slippery, the first member 20 will easily slide into a woman's vagina without subjecting the internal tissues of the vagina to abrasion. The first member 20 can be coated to give it a high slip characteristic. Wax, polyethylene, a combination of wax and polyethylene, cellophane and clay are representative coatings that can be applied to the first member 20 to facilitate comfortable insertion.

The first member 20 can be a straight, elongated cylindrical tube formed on a central longitudinal axis X--X. It is also possible to form the first member 20 into an arcuate shape. The arcuate or curved shape can assist in providing comfort when inserting the first member 20 into a woman's vagina. With a curved tampon applicator, it is possible to employ a curved tampon which again may be more comfortable for some women to use since the shape of the tampon may better fit the curvature of a woman's vagina.

The first member 20 has first and second spaced apart ends 28 and 30, respectively. The first member 20 can also have either a constant outer diameter or a stepped outer profile. Preferably, the first member 20 will have an essentially constant diameter over a major portion of it's length. Integrally formed on the first end 28 of the first member 20 and extending outwardly therefrom is an insertion tip 32. The insertion tip 32 is designed to facilitate insertion of the first member 20 into a woman's vagina in a comfortable manner. The insertion tip 32 includes a semi-spherical shaped portion 34 and a frusto-conical shaped portion 36. The frusto-conical shaped portion 36 is situated between the semi-spherical shaped portion 34 and the first member 20. The frusto-conical shaped portion 36 tapers inward toward the longitudinal central axis X--X as it approaches the semi-spherical shaped portion 34. The inward taper is at an angle of between about 5° to about 40° relative to the exterior surface 26 of the first member 20. Preferably, the angle is between about 5° to about 25°, and most preferably, the angle is between about 5° to about 15°. The frusto-conical shaped portion 36 has an outside diameter at one end which is approximately equal to the outside diameter of the first member 20 and has an outside diameter at an opposite end which is approximately equal to the outside diameter of the semi-spherical shaped portion 34.

The length of the frusto-conical shaped portion 36 can be as long as 1 inch (25.4 mm). Preferably, the length of the frusto-conical shaped portion 36 is less than about .75 inches (about 19 mm), and most preferably, between about .12 inches (about 3.1 mm) to about .5 inches (about 12.7 mm). The frusto-conical shaped portion 36 provides a smooth, gradual transition from the semi-spherical shaped portion 34 to the first member 20 and therefore facilitates comfortable insertion of the tampon applicator 10 into a woman's vagina.

The semi-spherical shaped portion 34 is positioned on the leading or insertion end of the tampon applicator 10. The semi-spherical shaped portion 34 has a diameter which is only a fraction of the outside diameter of the first member 20. For example, the diameter of the semi-spherical shaped portion 34 can range between about 50% to about 90% of the outside diameter of the first member 20.

The insertion tip 32 has a wall 38 having a thickness which is approximately equal to the thickness of the wall 24 which forms the first member 20. However, it is possible to construct the wall 38 so that it has a thickness which is less than or greater than the thickness of the wall 24, if desired.

Referring to Fig. 4, the insertion tip 32 is shown in cross-section with the frusto-conical shaped portion 36 extending outward away from the first end 28 of the first member 20. The frusto-conical shaped portion 36 terminates into the semi-spherical shaped portion 34. The cross-section of the semi-spherical shaped portion 34 spans an arc (A) of approximately 180 degrees. The semi-spherical shaped portion 34 is formed on a diameter which is sized to be equal or slightly smaller than the smallest diameter of the frusto-conical shaped portion 36. For example, if the smallest outside diameter of the frusto-conical shaped portion 36 is about .40 inches (about 10.0 mm), the semi-spherical shaped portion 34 can be formed on a diameter of about .40 inches (about 10.0 mm). This means that the smallest diameter of the frusto-conical shaped portion 36 and the diameter of the semi-spherical shaped portion 34 will be equal and this measurement will be equal to a fraction of the outside diameter of the first member 20.

A relatively small aperture 40 is formed in the center of the semi-spherical shaped portion 34 and is coaxially aligned with the longitudinal axis X--X of the first member 20. The aperture 40 can have a diameter of at least about 1.5 mm, preferably between about 1.5 to about 5.0 mm, and more preferably, between about 3.0 to about 3.5 mm. Another way of sizing the diameter of the aperture 40 is to make it less than about 30% of the diameter of the first member 20, preferably, between about 10% to about 30% of the diameter of the first member 20, and most preferably, less than about 20% of the diameter of the first member 20. It should be noted that although the aperture 40 is described as a circle, it is possible to form the aperture 40 in other shapes such as a polygon, a square, a pentagon, a hexagon, an octagon, etc. The small aperture 40 should extend through the semi-spherical shaped portion 34 of the insertion tip 32 and have a side wall 42 which is aligned essentially parallel to the longitudinal axis X--X. In addition, the aperture 40 can be rounded or contain a radius 44 on it's exterior surface to assure that no sharp edges are present which could pinch or cut the sensitive tissues of a woman's vagina. The purpose of the small aperture 40 in the end of the insertion tip 32 is to facilitate the subsequent unfolding of the pleats during use, as will be described below. The aperture 40 also assures that the pleats will symmetrically open about the longitudinal axis X--X of the first member 20. A further benefit of the aperture 40 is that it provides a visual means for the user to inspect the tampon applicator 10 and assure herself that a tampon 12 is present in the first member 20.

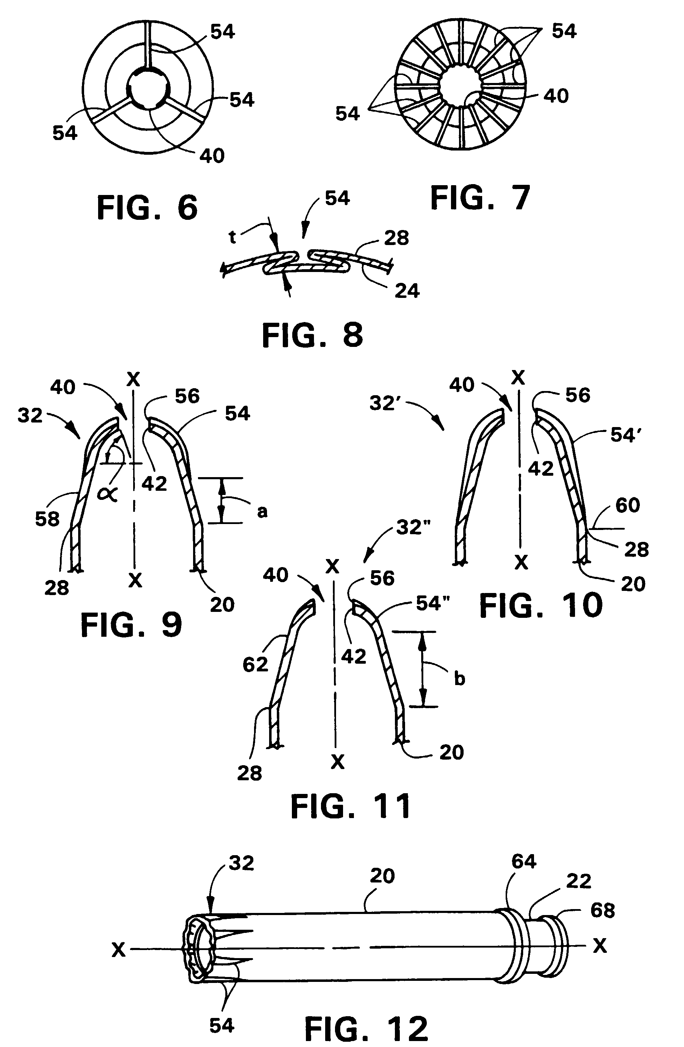

The design in Fig. 4 is to be contrasted to the embodiment shown in Fig. 5, which is not an embodiment according to the invention. In Fig. 5 an enlarged aperture 46 is depicted having a side wall 48 which tapers downward and inward to form a sharp point 50 adjacent to an interior surface 52 of the insertion tip 32. The sharp point 50 is more likely to pinch or trap vaginal tissue and therefore could cause discomfort during insertion. In addition, the larger diameter of the aperture 46 exposes a greater area of the absorbent tampon 12 and this could cause abrasion with the vaginal tissues during insertion. The embodiment shown in Fig. 4 is more desirable for comfort.

Referring again to Fig. 4, the configuration of the aperture 40 is preferred for it is smaller in diameter and therefore exposes a smaller amount of the absorbent tampon 12. Since a tampon is normally dry and consists of a plurality of absorbent fibers, it can cause abrasion against the walls of a woman's vagina as it is being inserted. By reducing the amount of surface area of the tampon 12 which is exposed to the vaginal tissue, one can decrease the discomfort during the insertion process. In addition, since the insertion tip 32 is essentially closed, it also lowers the frictional force between the exterior surface 26 of the tampon applicator 10 and the walls of the vagina. Furthermore, the small diameter of the aperture 40 also decreases the possibility of trapping or pinching vaginal tissue therein.

Referring to Figs. 3, 6 and 7, the insertion tip 32 is shown having a plurality of pleats 54 which can open radially outward such that the insertion tip 32 has a diameter approximately equal to or greater than the diameter of the first member 20. Either an even or an odd number of pleats 54 can be present and the pleats 54 can be equally spaced apart or they can be non-uniformly arranged. Uniform pleats spaced an equal distance apart are preferred over a random arrangement. For ease of manufacturing, it is preferred that the pleats 54 be equally spaced relative to one another. Each pleat 54 is a fold formed by doubling the material upon itself and then pressing or adhering the material into place. Although eight equally spaced apart pleats 54 are shown in Fig. 3, it is possible to utilize various numbers of pleats 54. The number of pleats 54 can vary from between three to about thirty-two pleats, preferably between about 5 to about 16 pleats, and most preferably, between about 6 to about 12 pleats.

In Fig. 6, an embodiment is shown with three equally spaced pleats 54, while in Fig. 7, sixteen pleats 54 are displayed. The minimum number of pleats 54 should be no less than three because the force required to open the insertion tip 32 normally increases as the number of pleats 54 decrease. If the force becomes too large, the tampon applicator 10 could bend or deform during the insertion process and this may cause discomfort. When more than thirty-two pleats 54 are used, the expulsion force may be lowered but it becomes difficult to form so many pleats on the insertion tip 32.

Referring to Fig. 8, a schematic view of a pleat 54 is shown. The pleat 54 is obtained by folding the paper, paperboard, cardboard material upon itself so that when the pleat 54 is opened or unfolded it will occupy a much larger surface area. The thickness of the material forming the insertion tip 32 can be equal to or slightly less than the thickness of the first member 20. For the first member 20, a thickness of about .1 mm to about .7 mm is satisfactory with a thickness of about .25 mm to about .5 mm being preferred. The insertion tip 32 can have a thickness between about .1 mm to about .7 mm. In the folded condition, the pleat 54 has a thickness, indicated by the letter "t" of less than about 0.7 mm, preferably between about .25 mm to about .35 mm. Another way of stating this is to say that the thickness of the pleat 54, in the folded condition, will be greater than twice the thickness of the material from which the insertion tip 32 is constructed. One particular apparatus and method for crimping, pleating and forming a tip on a hollow tube is taught in co-pending U.S. Serial No. 08/300,987 filed September 6, 1994 which is incorporated by reference and made a part hereof.

Referring to Figs. 9-11, three different embodiments of a pleat are depicted. In Fig. 9, the pleat 54 is depicted as having a first end 56 which coincides with the side wall 42 of the aperture 40. This means that the first end 56 of the pleat 54 forms a portion of the arc of the aperture 40. The pleat 54 also has a second end 58 which coincides with a point located on the outer circumference of the frusto-conical shaped portion 36. This point is spaced a distance "a" from the location where the frusto-conical shaped portion 36 is integrally joined to the first member 20. By forming the pleat 54 with this particular length, one can control the amount of force needed to open the insertion tip 32 and push the tampon 12 therethrough. Usually, a lower force is required to open the pleats when each pleat 54 has a length which extends into the outer circumference of the frusto-conical shaped portion 36.

In Fig. 9, the semi-spherical shaped portion 34 spans a radial arc, identified as angle alpha (α), which extends from the first end 56 to the point where the semi-spherical shaped portion 34 is integrally joined to the frusto-conical shaped portion 36. The angle alpha (α) is between about 60° to about 90°, preferably between about 75° to about 90°, and most preferably, greater than 80'. The angle alpha (α) would be 90° if the aperture 40 was not present. The size of the aperture 40 will partially determine the exact angle of the semi-spherical shaped portion 34. The angle alpha (α) should be as close to 90° as possible without completely enclosing the forward end of the tampon 12.

In Fig. 10, an alternative embodiment of an insertion tip 32' is depicted wherein a pleat 54' is shown having a first end 56 which coincides with the side wall 42 of the aperture 40. In other words, the first end 56 of the pleat 54' forms a portion of the arc of the aperture 40. The pleat 54' also has a second end 60 which coincides with the point where the frusto-conical shaped portion 36 is integrally joined to the first end 28 of the first member 20. By forming the pleat 54' with this particular length, one can control the amount of force needed to open the insertion tip 32' and push the tampon 12 therethrough. Since the length of each pleat 54', shown in Fig. 10, is slightly longer than the length of each pleat 54, shown in Fig. 9, the force required to open the pleats 54' may be slightly less.

In Fig. 11, a third embodiment of an insertion tip 32" is depicted wherein a pleat 54" is shown having a first end 56 which coincides with the side wall 42 of the aperture 40. In other words, the first end 56 of the pleat 54" forms a portion of the arc of the aperture 40. The pleat 54" also has a second end 62 which coincides with the point where the semi-spherical shaped portion 34 is integrally joined to the frusto-conical shaped portion 36. This point is spaced a distance "b" from the location where the frusto-conical shaped portion 36 is integrally joined to the first end 28 of the first member 20. By forming the pleat 54" with this particular length, one can control the amount of force needed to open the insertion tip 32" and push the tampon 12 therethrough. Although the force required to open the pleats 54" may be greater than the force required with the designs shown in Figs. 9 and 10, the force is still within acceptable limits.

It should be noted that both the length and diameter of commercially available tampons do vary and therefore the tampon applicators 10 should be manufactured in a variety of sizes. Tampons can vary in length from about 1 to about 3 inches (about 25.4 mm to about 76.2 mm) but preferably are about 2 inches (about 50.8 mm) in length. The tampon diameter will also vary from about .25 inches to about .75 inches (about 6.4 mm to about 19.0 mm). In addition, the material from which the tampon 12 is constructed, the smoothness of the internal surface of the first member 20, the shape of the second member 22, etc. all contribute to establish a needed expulsion force to open and expel the tampon 12. This force should range from between about 250 grams to about 1,500 grams, preferably less than about 1,200 grams, and most preferably, less than about 1,000 grams. A lower force value is preferred for it assures that the tampon applicator 10 will be less susceptible to being bent or deformed as the tampon 12 is expelled. A bent applicator could cause the tampon to be inserted incorrectly. A lower force value also makes the tampon applicator 10 easier to use.

Referring again to Figs. 1 and 2, the first member 20 can have a fingergrip ring 64 located approximate the second end 30. The fingergrip ring 64 can be integrally formed from the material from which the first member 20 is constructed or it can be a separate member which is secured in place by an adhesive or some other type of attachment mechanism. The fingergrip ring 64 functions to provide a means for the user to grip the first member 20 and hold it between her thumb and middle finger. The user can then position her forefinger on the free end of the second member 22 and orient the first member 20 relative to her vagina while she pushes the second member 22 into the first member 20.

As stated above, the tampon applicator 10 includes a second member 22, also commonly referred to as an inner tube. The second member 22, like the first member 20, can be a spirally wound, a convolutely wound, or a longitudinally seamed, hollow tube constructed from paper, paperboard, cardboard, etc. The second member 22 can be constructed of the same material as the first member 20 or it can be made out of a different material. Furthermore, the second member 22 could be constructed as a laminate having two or more plies which are then spirally wound, convolutely wound or longitudinally seamed into a cylindrical tube. Either a wound tube or a longitudinally seamed tube is preferred because the finished tube will have a wall 66 with a constant thickness. However, some manufacturers may prefer to construct the second member 22 as a solid stick or use some other unique shape. It is also possible to form a fingergrip ring or flange 68 on the outer end of the second member 22 to provide an enlarged surface onto which the user's forefinger can rest. The fingergrip ring 68 thereby functions as a seat for the forefinger and facilitates movement of the second member 22 into the first member 20.

Referring to Fig. 12, the second member 22 functions by being telescopically movable relative to the first member 20. As the second member 22 is pushed into the first member 20, the tampon 12 is forced forward against the pleats 54. The contact by the tampon 12 causes the pleats 54 to open radially outward to a diameter which is sufficient to allow the tampon 12 to be expelled from the first member 20. The open arrangement of the pleats 54 is shown in Fig. 12 after the tampon 12 has been expelled. With the tampon 12 positioned in the woman's vagina, the tampon applicator 10 is withdrawn and properly discarded.

The tampon applicator 10 having the improved pleated tip 32 works well in combination with a catamenial tampon having a shaped nose. This is especially true when the shaped nose on the tampon 12 is configured to conform to the interior surface 52 of the insertion tip 32. For example, the tampon 12 can be configured to have a generally rounded forward end which conforms to the interior surface of the semi-spherical shaped portion 34 or alternatively, the tampon 12 can be configured to have a frusto-conical shape with a rounded forward end which conforms to the interior surface of the entire insertion tip 32.

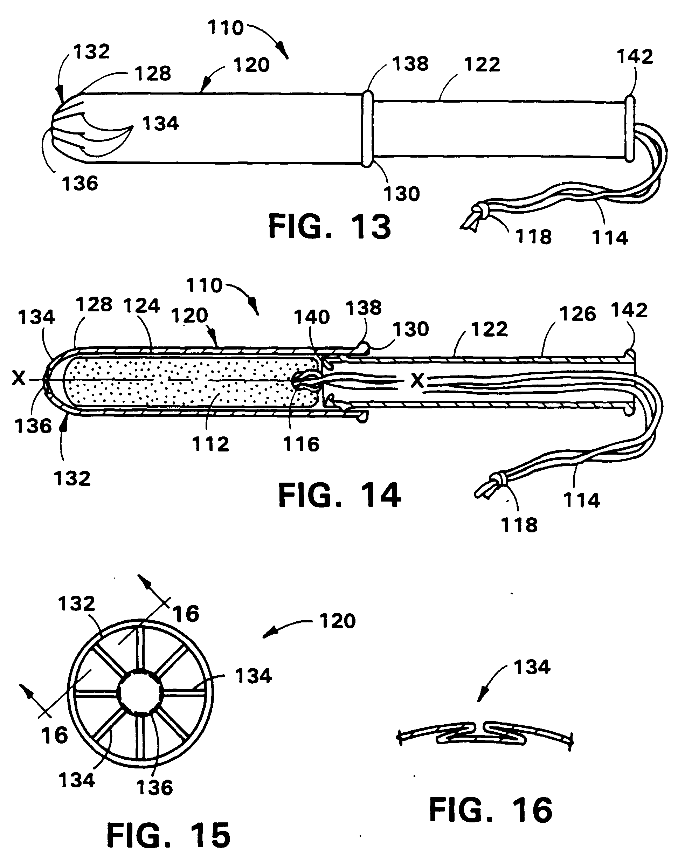

Referring to Figs. 13-15, a tampon applicator 110 is shown which is designed to house an absorbent tampon 112 and provide a comfortable means of inserting the tampon 112 into a woman's vagina. A tampon is an absorbent member primarily designed to be worn by a woman during her menstrual period to absorb menses, blood and other body fluid. The tampon 112 can be made from natural or synthetic fibers including cellulose fibers such as cotton or rayon, or artificial fibers such as polyester, polypropylene, nylon or blends thereof. Other types of fibers may also be used, such as cellulose sponge or a sponge formed from elastomeric materials. A blend of cotton and rayon fibers works well.

The tampon 112 is normally compressed into the form of a cylinder and can have a blunt, rounded or shaped forward end. The tampon 112 commonly has a withdrawal string 114 fastened to an end thereof which serves as a means for withdrawing the soiled tampon from the woman's vagina. The withdrawal string 114 is permanently affixed to the tampon 112, for example, by looping it through an aperture 116 formed transversely through the tampon 112. In addition, the withdrawal string 114 can have a knot 118 formed at it's free end to assure that the string 114 will not separate from the tampon 112.

The tampon applicator 110 includes an outer tube 120 and an inner tube 122. The outer tube 120 is preferably in the form of a spirally wound, convolutely wound or longitudinally seamed, hollow tube which is formed from paper, paperboard, cardboard or a combination thereof. The inner tube 122 can be formed from the same material as the outer tube 120, or alternatively, be made of a different material. The inner tube 122 should have a constant external diameter so as to easily slide within the inner diameter of the outer tube 120. It is also possible to construct the inner tube 122 as a solid stick, or use some other unique shape, which attaches directly to the tampon 112.

Both the outer tube 120 and the inner tube 122 are fairly rigid and commonly have a diameter of about 10 mm to about 20 mm, with the inner tube 122 being slightly smaller in diameter than the outer tube 120. The outer tube 120 has a wall 124 with a predetermined thickness of about .2 mm to about .6 mm. The inner tube has a wall 126 which is slightly thinner. The walls 124 and 126 can be constructed from a single ply of material or be formed from two or more plies which are bonded together to form a laminate. The use of two or more plies or layers is preferred for it enables the manufacturer to use certain material in the various layers which can enhance the performance of the tampon applicator 110. When two or more layers are utilized, all the layers can be spirally wound, convolutely wound or longitudinally seamed to form an elongated cylinder. The exterior surface of the wall 124 can be constructed using a smooth thin layer of material to facilitate insertion of the first member 120 into a woman's vagina.

The layers forming the walls 124 and 126 can be held together by an adhesive, such as glue, or by heat, pressure, ultrasonics, etc. The adhesive can be either water-soluble or water-insoluble. A water-soluble adhesive is preferred for environmental reasons in that the tubes 120 and 122 will quickly break apart when immersed in water. Such immersion will occur should the tubes 120 and 122 be disposed of by flushing them down a toilet. Exposure of the tubes 120 and 122 to a municipal's waste treatment plant wherein soaking in water, interaction with chemicals and agitation all occur, will cause the tubes 120 and 122 to break apart in a relatively short period of time.

The outer tube 120 is sized and configured to house the absorbent tampon 112 and the inner tube 122 is sized and configured to push the tampon 112 out of the outer tube 120. The outer tube 120 can be a straight, elongated cylindrical tube formed on a central longitudinal axis X--X. It is also possible to form the outer tube 120 into an arcuate shape. The arcuate or curved shape can assist in providing comfort when inserting the outer tube 120 into a woman's vagina. The inner tube 122 should be configured to telescopically slide in the outer tube 120. With a curved tampon applicator, it is possible to employ a curved tampon which again may be more comfortable for some women to use since the shape of the tampon may better fit the curvature of a woman's vagina.

The outer tube 120 has first and second spaced apart ends 128 and 130, respectively. The outer tube 120 can also have either a constant outer diameter or a stepped outer profile. Preferably, the outer tube 120 will have an essentially constant diameter over a major portion of it's length. Integrally formed on the first end 128 of the outer tube 120 and extending outwardly therefrom is an insertion tip 132. The insertion tip 132 is designed to facilitate insertion of the outer tube 120 into a woman's vagina in a comfortable manner. The insertion tip 132 contains a number of pleats 134 and has a semi-spherical configuration with a diameter which is approximately equal to the outside diameter of the outer tube 120. The pleats 134 can be uniformly spaced apart or they can be randomly arranged. The insertion tip 132 can have the same thickness as the outer tube 120 or be made thinner or thicker, if desired.

An aperture 136 is formed in the center of the semi-spherical shaped insertion tip 132 and is coaxially aligned with the longitudinal axis X--X. The aperture 136 can have a diameter of at least about 1.5 mm, preferably between about 1.5 to about 5.0 mm, and more preferably, between about 3.0 to about 3.5 mm. Another way of sizing the diameter of the aperture 136 is to make it less than about 30% of the diameter of the outer tube 120, preferably, between about 10% to about 30%, and most preferably, less than about 20% of the diameter of the outer tube 120. It should be noted that although the aperture 136 is described as a circle, it is possible to form the aperture 136 in other shapes such as a polygon, a square, a pentagon, a hexagon, an octagon, etc. The aperture 136 should extend entirely through the insertion tip 132. The purpose of the aperture 136 in the end of the insertion tip 132 is to facilitate the subsequent unfolding of the pleats 134 during use. The aperture 136 also assures that the pleats 134 will symmetrically open about the longitudinal axis X--X of the outer tube 120. A further benefit of the aperture 136 is that it provides a visual means for the user to inspect the tampon applicator 110 and assure herself that a tampon 112 is present in the outer tube 120.

With the aperture 136 being small, less of the absorbent tampon 112 is exposed to the vaginal tissue when the tampon applicator 110 is inserted into the woman's vagina. Since a tampon 112 is normally dry and consists of a plurality of absorbent fibers, it can cause abrasion against the walls of a woman's vagina as it is being inserted. By reducing the amount of surface area of the tampon 112 which is exposed to the vaginal tissue, one can decrease the discomfort during the insertion process. In addition, since a majority of the insertion tip 132 is closed, the frictional force between the exterior surface 126 of the outer tube 120 and the walls of the vagina is reduced. Furthermore, the small diameter of the aperture 136 also decreases the possibility of trapping or pinching vaginal tissue therein.

Referring to Figs. 15 and 16, the insertion tip 132 is shown having a plurality of pleats 134 which can radially open such that the insertion tip 132 has a diameter which is approximately equal to or larger than the diameter of the outer tube 120. The term "pleat" as used herein refers to material which is folded upon itself, for example, by doubling the material upon itself and then pressing it into place. A representative view of a pleat 134 is depicted in Fig. 16. Either an even or an odd number of pleats 34 can be present and the pleats 34 can be equally spaced apart or they can be uniformly or randomly arranged. For ease of manufacturing, it is preferred that the pleats 34 be equally spaced relative to one another. Each pleat 34 is formed by doubling the material upon itself and then pressing or adhering the material into place. Although eight equally spaced apart pleats 34 are shown in Fig. 15, it is possible to utilize various numbers of pleats 34. The number of pleats 34 can vary from between three to about thirty-two pleats, preferably between five to sixteen pleats, and most preferably, eight pleats.

Referring again to Figs. 13 and 14, the outer tube 120 can have a fingergrip ring 138 located approximate the second end 130. The fingergrip ring 138 can be integrally formed from the material from which the outer tube 120 is constructed or it can be a separate member which is secured in place by an adhesive or some other type of attachment mechanism. The fingergrip ring 138 functions to provide a means for the user to grip the outer tube 120 and hold it between her thumb and middle finger. The user can then position her forefinger on the free end of the inner tube 122 and orient the outer tube 120 relative to her vagina while she pushes the inner tube 122 into the outer tube 120.

The inner tube 122 can have an inwardly directed flange 140 formed at its forward end which provides an enlarged surface for contacting the rear end of the tampon 112. The inner tube 122 can also have a radial, outwardly extending ring 142 formed adjacent to the outer or free end of the inner tube 122 which provides an enlarged surface onto which the user's forefinger can rest. The ring 142 thereby functions as a seat for the forefinger and facilitates movement of the inner tube 122 into the outer tube 120.

The inner tube 122 functions by being telescopically movable relative to the outer tube 120. As the inner tube 122 is pushed into the outer tube 120, the tampon 112 is forced forward against the pleats 134. The contact by the tampon 112 causes the pleats 134 to radially open to a diameter which is sufficient to allow the tampon 112 to be expelled from the outer tube 120. With the tampon 112 properly positioned in the woman's vagina, the tampon applicator 110 is withdrawn and properly discarded.

### APPARATUS

The outer tube 120 can have the insertion tip 132 formed into the desired semi-spherical configuration with the central aperture 136 by using the apparatus described below.

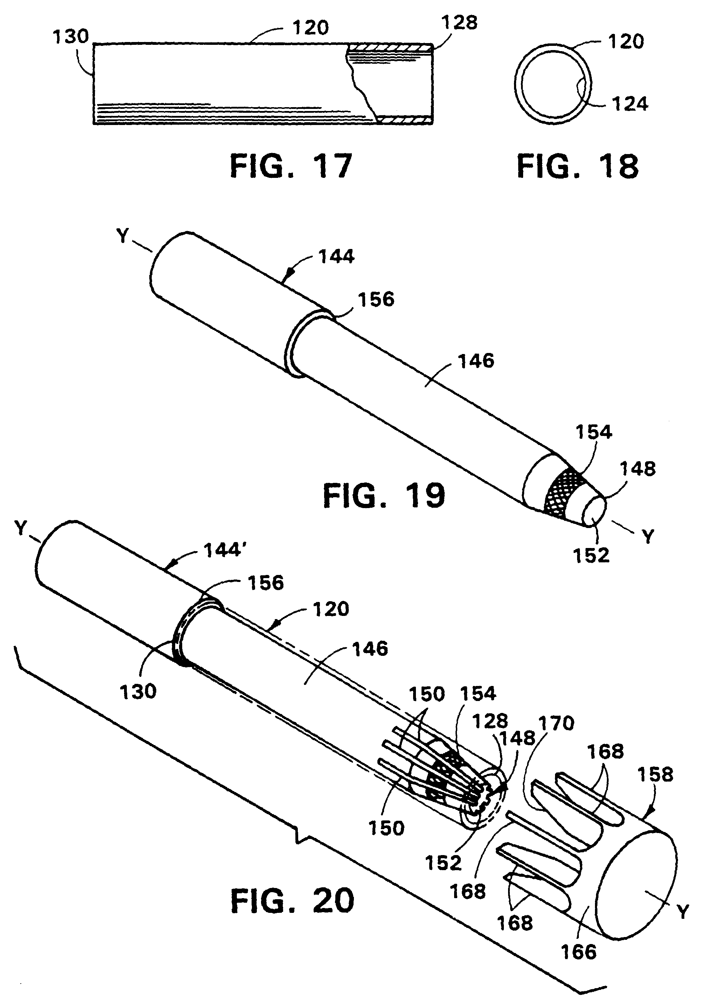

Referring to Figs. 17 and 18, the outer tube 120 is shown before the insertion tip 132 is formed. At this stage, the outer tube 120 has an essentially constant inside diameter and the wall 124 has a constant thickness.

Referring to Figs. 19 and 20, a first punch 144 is shown having a tubular section 146 which is sized and configured to receive the outer tube 120. In other words, the outer tube 120 must be able to slide onto the tubular section 146 with only a small amount of clearance therebetween. The first punch 144 has a tip 148. The tip 148 can be smooth or void of any grooves as shown in Fig. 19. Alternatively, the first punch 144' can have a configured tip 148 with a plurality of elongated grooves 150 formed therein, as is depicted in Fig. 20. When the grooves 150 are present, there should be at least four grooves 150, preferably between eight to twelve grooves 150, with eight grooves 150 being most preferred. The purpose of the grooves 150 will be explained shortly.

The tip 148 can be formed into a frusto-conical shape having a blunt end 152. Other shapes can also be utilized if desired. At least a portion of the exterior surface of the tip 148 can be knurled 154 to provide a frictional surface between the tip 148 and the inner surface of the outer tube 120 as the insertion tip 132 is being formed. A medium knurl 154 will provide an adequate frictional surface for the crimping operation. The first punch 144 or 144' also has a shoulder 156 formed at an opposite end of the tubular section 146 which acts as a stop for the outer tube 120. It should be noted that the length of the tubular section 146 is sized to conform closely to the length of the outer tube 120. A typical outer tube 120 will have a length of between about 2 inches to about 4 inches (about 50.8 mm to about 101.6 mm), preferably about 3 inches to about 3.5 inches (about 76.2 mm to about 88.9 mm), most preferably, at least about 3.12 inches (about 79.2 mm). The tubular section 146 should have a length which is equal to or slightly greater than the initial length of the outer tube 120 as shown in Fig. 17. The first end 128 of the tube 120 can be aligned approximately flush with the tip 148 when the tube 120 is positioned on the tubular section 146. However, an extra length of about 0.06 inches (about 1.5 mm) on the tubular section 146 of the first punch 144 or 144' is advantageous for permitting the first punch 144 or 144' to mate with a first die 158.

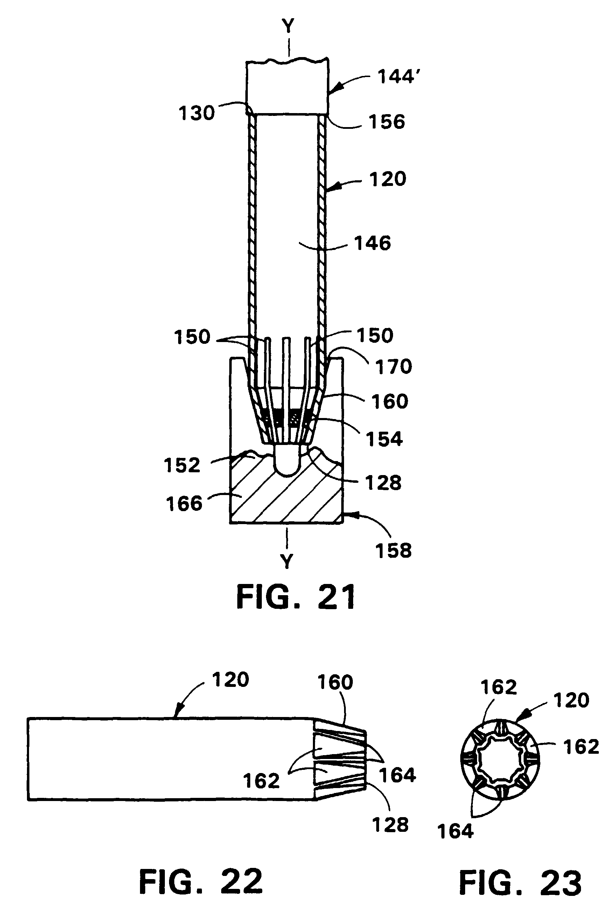

Referring to Figs. 20 and 21, the first punch 144' is shown with the outer tube 120 slid onto the tubular section 146 such that the second end 130 of the outer tube 120 abuts the shoulder 156 and is mateable with the first die 158. The axial engagement of both the first punch 144' and the outer tube 120 with the first die 158 enables the tip 132 of the outer tube 120 to be crimped. The term "crimped" as used herein refers to pressing or pinching the material forming the insertion tip 132 into small, regular folds or ridges with troughs therebetween.

Referring to Figs. 22 and 23, the crimped tip 160 consists of a plurality of ridges 162 and troughs 164 formed about the circumference of the first end 128 of the outer tube 120. The troughs 164 are the deepest adjacent the first end 128 and become shallower as the troughs move away from the first end 128. The ridges 162 are formed on a circle having a smaller diameter adjacent the first end 128 and expand outward as the ridges 162 move away from the first end 128.

Referring again to Figs. 20 and 21, the crimped tip 160 is formed by the engagement of the first punch 144 or 144' with the first die 158. The first die 158 includes a base 166 having a plurality of blades 168 extending axially outward therefrom. There should be at least four blades 168, preferably, between eight to twelve blades, with eight blades being the most preferred. For best results with hard board papers, each groove 150 formed in the first punch 144' should align with a blade 168 formed in the first die 158. Either an even number or an odd number of blades 168 can be utilized. An even number of blades 168 are easier to machine and the first die 158 will then have a symmetrical shape, which is also advantageous. For example, symmetrically shaped dies can be measured across their tips to determine their size.

The blades 168 can range from about .5 inches (about 12.7 mm) to about 2 inches (about 50.8 mm) in length. A length of approximately 1 inch (25.4 mm) is sufficient. The blades 168 have an angled or tapered inner surface 170 which enables then to mate with the smooth tip 148 formed on the first punch 144 or mate with and axially enter a corresponding groove 150 formed in the first punch 144'. The angle can vary depending upon the taper on the smooth tip 148 or depending on the angle at which each corresponding groove 150 is formed. The angle on each blade 168 can be equal to or different from the angle formed on the smooth tip 148 and can also be equal to or different from the angle to which each groove 150 is machined. When the grooves 150 are present, each groove 150 should be sized to be larger than the corresponding blade 168 so that the thickness of wall 124 of the outer tube 120 can also be received into the grooves 150. Each blade 168 is machined to an angle which is different from the angle to which the bottom of the grooves 150 have been machined to. When the first punch 144' is fully inserted into the first die 158, the angled surfaces 170 of each blade 168 is spaced apart from the bottom of each corresponding groove 150. This clearance permits the wall thickness of the outer tube 120 to be sandwiched therebetween and provides the undulating surface which is the crimp 160 shown in Fig. 23. The angle that each groove 150 and each blade 168 is formed at can vary. The grooves 150 and the blades 168 can be formed at identical angles relative to a longitudinal central axis Y--Y of the first punch 144' and the first die 158, or they can be formed at different angles relative to one another. For outer tubes 120 formed from hard paperboard, good quality pleats 134 can be formed using the first punch 144 with the smooth tip 148 mating with the first die 158. For softer paperboard, it is advantageous to machine an angle of about 20° to the Y--Y axis in the first die 158 and to machine an angle of about 15° to the Y--Y axis in the first punch 144'.

Referring to Fig. 21, one can see the position of the outer tube 120 on the first punch 144' while the first end 128 is crimped. The knurled surface 154 serves to prevent the outer tube 120 from moving toward the shoulder 156 when the first punch 144' engages the first die 158. It has been found that the force exerted on the outer tube 120 increases as the first punch 144 or 144' engages deeper and deeper into the first die 158. When the knurled surface 154 is not present, this force can drive the outer tube 120 up against the shoulder 156 and cause the second end 130 of the outer tube 120 to become wrinkled and radially enlarged. Such a feature is not aesthetically pleasing and must be avoided.

After the outer tube 120 has a crimped tip 160 formed on the first end 128 thereof, the first punch 144 or 144' and the first die 158 are separated and the outer tube 120 is removed.

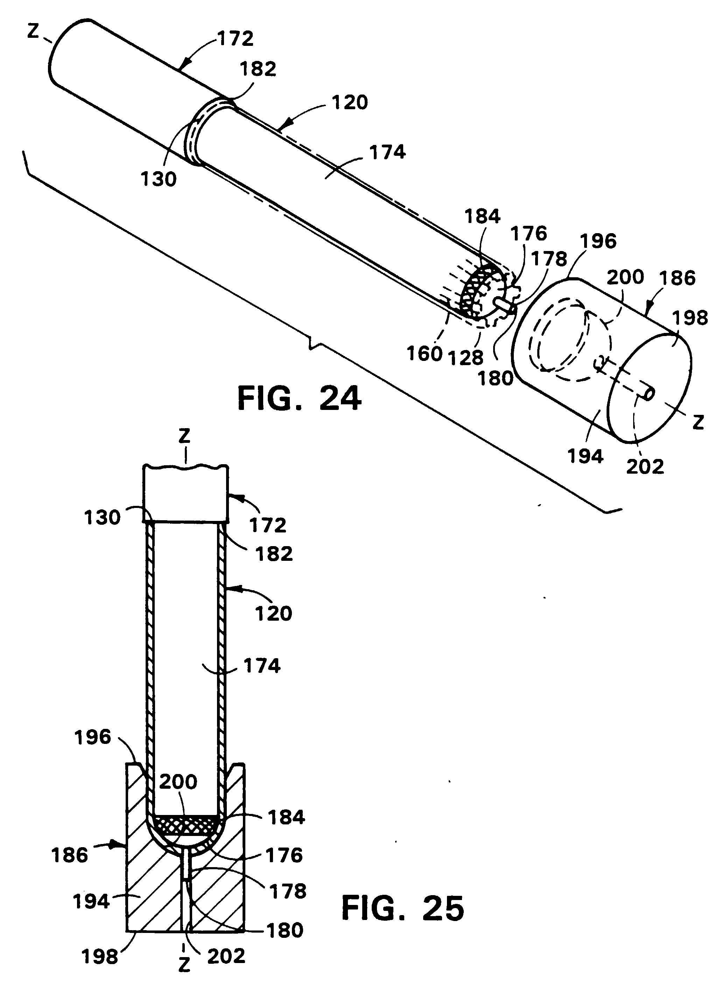

Referring to Figs. 24 and 25, the outer tube 120 is then subjected to a second operation wherein the crimped tip 160 is pleated and pressed into a semi-spherical configuration. This is accomplished by using a second punch 172 having a tubular section 174 which is sized to receive the outer tube 120. The second punch 172 has a semi-spherically shaped tip 176 with a pin 178 extending outward from the apex of the tip 176. The pin 178 has a distal or free end 180. The second punch 172 also contains a shoulder 182 formed at an opposite end of the tubular section 174 which acts as a stop for the outer tube 120. It should be noted that the length of the tubular section 174 is sized to conform closely to the length of the outer tube 120. The second punch 172 is designed to have the outer tube 120 slid over the tubular section 174 until the second end 130 of the outer tube 120 abuts against the shoulder 182. In this position, the crimped tip 160 formed on the first end 128 of the outer tube 120 should extend about 0.06 inches to about 0.12 inches (about 1.5 mm to about 3.0 mm) beyond the apex of the semi-spherically shaped tip 176. The free end 180 of the pin 178, however, will extend beyond the crimped end 160 by at least 0.06 inches (1.5 mm), and preferably more.

At least a portion of the semi-spherically shaped tip 176 can be knurled 184 to provide a frictional surface between the tip 176 and the inner surface of the outer tube 120 as the insertion tip 132 is being formed. A medium knurl 184 will provide an adequate frictional surface for the pleating and forming operation. The knurled surface 184 serves to prevent the outer tube 120 from moving toward the shoulder 182 of the second punch 172.

The pin 178 can have a length of at least 0.06 inches (1.5 mm) but is preferably longer. The pin 178 should have a diameter of at least 0.062 inches (1.5 mm), preferably at least 0.125 inches (3.1 mm), and can be larger if desired. The cross-section of the pin 178 is preferably circular but could be of a different configuration if desired. A circular cross-section is preferred for it forms an opening with a circular periphery. A circular opening is aesthetically pleasing to the eye and since one of the purposes of the aperture 136 is to allow the consumer to see if a tampon 112 is present in the tampon applicator 110, the aperture could be circular. The pin 178 is shown having an essentially constant outside diameter. However, it is possible to form the pin 178 such that it tapers down in diameter from a larger diameter located adjacent to the point of attachment to the apex of the semi-spherical tip 176 to a smaller diameter adjacent the free end 180.

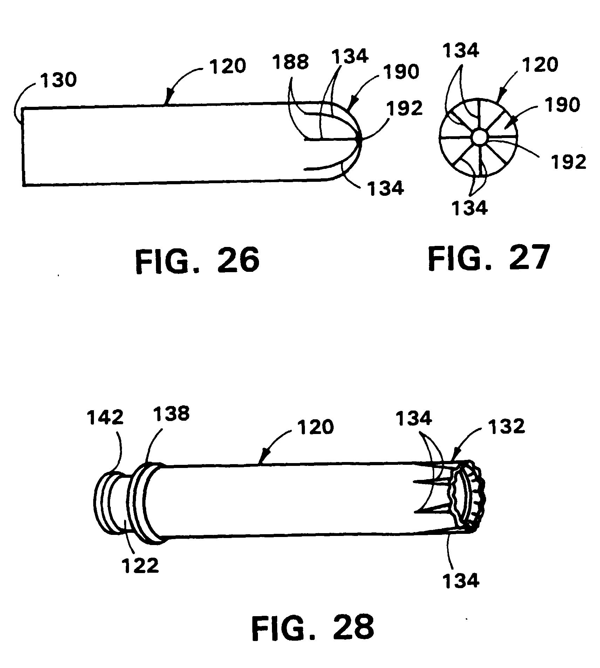

The second punch 172 is sized and configured to engage with a second die 186 so as to transform the crimped tip 160 of the outer tube 120 into a plurality of pleats 134 and form the pleats 134 into a semi-spherically shaped tip 190, see Figs. 26 and 27. The pleats 134 can be uniformly or randomly spaced apart and can have a dovetail-like appearance. The length of each pleat 134 should be aligned approximately straight with or parallel to the longitudinal axis of the tube 120 versus being undulating or curved. A straight pleat normally requires a lesser amount of force to open. The pleats 134 should terminate at a point 188 which is approximately tangent to the point where the semi-spherically shaped tip 190 joins to the exterior surface of the outer tube 120. The semi-spherically shaped tip 190 will have a central aperture 192 formed therethrough because of the presence of the pin 178. The aperture 192 allows the consumer to visually inspect the tampon applicator 10 to see if a tampon 112 is present. The aperture 192 can vary with the diameter of the tube 120 but should not be so large that it would allow a woman to feel it as she inserts the tampon 112 into her vagina. If the aperture 192 is too large, it could cause discomfort as the woman inserts the tampon applicator 110 into her vagina.

Referring again to Figs. 24 and 25, the second die 186 includes a base 194 having a first end 196 and a second end 198. A semi-spherically shaped cavity 200 formed in the base 194 adjacent to the first end 196. The semi-spherically shaped cavity 200 is sized to receive the semi-spherically shaped tip 176 formed on the outer tube 120 as well as the wall thickness of the outer tube 120. This difference in size will allow the insertion tip 132 to be formed on the first end 128 of the outer tube 120. The surface of the cavity 200 is preferably polished to improve the appearance of the finished semi-spherically shaped tip 176 and to facilitate removal of the finished tube 120 from the second die 186. The polished surface can have a "surface roughness average" value of between about 0.1 µm to about 0.4 µm (about 4 micro inches to about 16 micro inches).

The base 194 also has a central passageway 202 formed therein which is axially aligned along a longitudinal central axis Z--Z. The passageway 202 extends from the bottom of the cavity 200 to the second end 198. If desired, the passageway 202 can be a closed passageway which terminates short of the second end 198. The passageway 202 is sized and configured to receive only the pin 178. The outside diameter of the pin 178 should be slightly smaller than the inside diameter of the passageway 202. The relationship between the mating second punch 172, the outer tube 120 and the second die 186 is clearly shown in Fig. 25.

It should be noted that both the length and diameter of commercially available tampons do vary and therefore the tampon applicator 110 should be manufactured in a variety of sizes. Tampons can vary in length from about 1 inch to about 3 inches (about 25.4 mm to about 76.2 mm) but preferably are about 2 inches (about 50.8 mm) in length. The tampon diameter will also vary from about .25 inches to about .75 inches (about 6.4 mm to about 19.0 mm). In addition, the material from which the tampon 112 is constructed, the smoothness of the internal surface of the outer tube 120, the shape of the inner tube 122, etc. all contribute to establish a needed expulsion force to open and expel the tampon 112. This force should range from between about 250 grams to about 1,500 grams, preferably less than about 1,200 grams, and most preferably, less than about 1,000 grams. A lower force value is preferred for it assures that the tampon applicator 110 will be less susceptible to being bent or deformed as the tampon 112 is expelled. A bent applicator could cause the tampon to be inserted incorrectly. A lower force value also makes the tampon applicator 110 easier to use. The size of the aperture 192 will also affect the amount of force needed to open the pleats 134. Typically, the larger the diameter of the aperture 192, the lower the force required to open the pleats 134.

### METHOD

The method of crimping, pleating and forming a semi-spherically shaped tip 190 on a hollow tube 120 is as follows, using the above-identified punches 144, 144' and 172, and dies 158 and 186. The above identified punches 144, 144' and 172, and dies 158 and 186 can be manually or automatically engaged and disengaged to form the insertion tip 132 on the outer tube 120. It is contemplated that the punches 144, 144' and 172, and the dies 158 and 186 will be actuated at sufficient speeds to crimp, pleat and form in excess of 100, preferably in excess of 300, and most preferably, more than 500 outer tubes per minute.

The method involves sliding the hollow tube 120 onto the first punch 144 or 144' until one end 130 of the tube 120 contacts the shoulder 156. The first punch 144 or 144' and the tube 120 are moved into engagement with the first die 158 and a plurality of crimps are formed on the opposite end 128 of the tube 120. The plurality of crimps form the crimped end 160. The first punch 144 or 144' is then disengaged from the first die 158 and the tube 120 having the crimped end 160 is removed from the first punch 144 or 144'. The tube 120 is then slid onto the second punch 172 until the non-crimped end 130 of the tube 120 contacts the shoulder 182. The second punch 172 and the tube 120 are brought into engagement with the second die 186 thereby allowing the axially extending pin 178 to enter the passageway 202. The mating of the second punch 172 with the second die 186 transforms the crimped end 160 of the tube 120 into a plurality of pleats 134 and forms the pleats 134 into a semi-spherically shaped tip 190 having a central aperture 192 formed therethrough. Once the tip 190 is formed, the second punch 172 is disengaged from the second die 186 and the outer tube 120 is removed from the second punch 172.

## Claims

1. A tampon applicator (10) comprising:
a) a first member (20) capable of housing an absorbent tampon (12), said first member (20) having a central longitudinal axis and first (28) and second ends (30);
b) an insertion tip (32) integrally formed on said first end (28) of said first member (20) and extending outwardly therefrom, said insertion tip (32) including a plurality of pleats (54) capable of expanding outward as said tampon (12) is expelled from said first member (20); and
c) a second member (22) telescopically mounted in said second end (30) of said first member (20), said second member (22) adapted to expel said tampon (12) through said insertion tip (32) as it is pushed into said first member (20), said tampon applicator (10) characterized in that said insertion tip (32) includes a semi-spherical shaped portion (34) and a frusto-conical shaped portion (36), said semi-spherical shaped portion (34) having an aperture (40) formed therethrough and said aperture (40) having a side wall (48) which is aligned essentially parallel to said central longitudinal axis, said aperture (40) having a diameter between 1.5 mm to 5.0 mm, and said frusto-conical shaped portion (36) is situated between said semi-spherical shaped portion (34) and said first end (28) of said first member (20) .

2. The tampon applicator (10) of claim 1, wherein said frusto-conical shaped portion (36) extends outward away from said first end (28) of said first member (20) and tapers inwardly at an angle of between about 5° to about 40°.

3. The tampon applicator (10) of claim 2, wherein said frusto-conical shaped portion (36) tapers inwardly at an angle of between about 5° to about 25°.

4. The tampon applicator (10) of claim 2 wherein said frusto-conical shaped portion (36) has a length of less than 2.54 cm (1 inch).

5. The tampon applicator (10) of claim 4, wherein said frusto-conical shaped portion (36) reduces in diameter from the point of attachment to said first end (28) of said first member (20) to the point of attachment to said semi-spherical shaped portion (34).

6. The tampon applicator (10) of claim 1, wherein said insertion tip (32) contains an even number of pleats (54).

7. The tampon applicator (10) of claim 1, wherein said insertion tip (32) contains an odd number of pleats (54).

8. The tampon applicator (10) of claim 1, wherein said insertion tip (32) contains from between three to thirty-two pleats (54) .

9. The tampon applicator (10) of claim 1, wherein said insertion tip (32) contains from between 6 to 12 pleats (54).

10. The tampon applicator (10) according to one of the preceding claims, wherein:
said first member (20) has a substantially smooth exterior surface, and said insertion tip (32) includes at least three pleats (54) capable of expanding radially outward as said tampon (12) is expelled from said first member (20).

11. The tampon applicator (10) of claim 10, wherein each of said pleats (54) has a first end (56) which coincides with said aperture (40) and a second end (58) which coincides with a point on an exterior surface of said frusto-conical shaped portion (36).

12. The tampon applicator (10) of claim 10, wherein each of said pleats (54) has a first end (56) which coincides with said aperture (40) and a second end (58) which coincides at a location where said frusto-conical shaped portion (36) is joined to said first member (20).

13. The tampon applicator (10) of claim 10, wherein each of said pleats (54) has a first end (56) which coincides with said aperture (40) and a second end (58) which coincides at a location where said semi-spherical shaped portion (34) is joined to said frusto-conical shaped portion (36).

14. The tampon applicator (10) of claim 10, wherein said frusto-conical shaped portion (36) has an outside diameter at one end which is approximately equal to the outside diameter of said first member (20).

15. The tampon applicator (10) of claim 14, wherein said frusto-conical shaped portion (36) has an outside diameter at an opposite end which is approximately equal to the outside diameter of said semi-spherical shaped portion (34).

16. The tampon applicator (10) according to claims 1 to 6 and 8 to 15, wherein said first member (20) is a hollow, spirally wound first member having a substantially smooth exterior surface and said insertion tip (32) includes eight pleats (54) spaced approximately an equal distance apart which are capable of expanding radially outwardly as said tampon (12) is expelled from said first member (20).

17. The tampon applicator (10) of claim 16, wherein said frusto-conical shaped portion (36) has an outside diameter at one end which is approximately equal to the outside diameter of said first member (20).

18. The tampon applicator (10) of claim 16, wherein said first member (20) has an outside diameter and said semi-spherical shaped portion (34) has a diameter which ranges between about 50% to about 90% of said outside diameter of said first member (20).

19. The tampon applicator (10) of claim 16, wherein said side wall (48) of said aperture (40) contains a radius formed adjacent to an exterior surface of said insertion tip (32).

20. The tampon applicator (10) of claim 16, wherein said pleats (54) have a thickness of less than 0.5 mm.

21. In combination, a tampon applicator (110) and a catamenial tampon (112) having a shaped nose, said combination comprising:
a) a first member (120) capable of housing said catamenial tampon (112), said first member (120) having a central longitudinal axis and first (128) and second ends (130);
b) an insertion tip (132) integrally formed on said first end (128) of said first member (120) and extending outwardly therefrom, said insertion tip including a plurality of pleats which are capable of expanding radially outward as said tampon is expelled from said first member; and
c) a second member (122) telescopically mounted in said second end (130) of said first member (120), said second member (122) adapted to expel said tampon (112) through said insertion tip (132) as it is pushed into said first member (120);
said combination characterized in that said insertion tip (132) of said tampon applicator (110) includes a semi-spherically shaped portion and a frusto-conical shaped portion, said semi-spherical shaped portion having an aperture (136) formed therethough and said aperture (136) having a side wall which is aligned essentially parallel to said central longitudinal axis, said aperture having a diameter between 1.5 mm to 5.0 mm, and said frusto-conical shaped portion situated between said semi-spherical shaped portion and said first end (128) of said first member (120).

22. The combination of claim 21, wherein said shaped nose on said tampon is configured to conform to said interior surface of said insertion tip.

23. An apparatus for crimping, pleating and forming a tip (132) on a hollow tube (120), said apparatus comprising:
a) a first punch (144) having a tubular section (146) sized to receive said tube (120) and a shoulder (156) formed at an opposite end of said tubular section (146) which acts as a stop for said tube (120);
b) a first die (158) mateable with said first punch (144) and tube (120) to crimp an end of said tube (120), said first die (158) including a base (166) having a plurality of blades (168) extending axially outward therefrom, each of said blades (168) capable of engaging said tube (120) and crimping the tip (132) of said tube positioned therebetween;
c) a second punch (172) having a tubular section (174) sized to receive said tube (120) and having a semi-spherically shaped tip (176) with a pin extending outward from the apex of said tip (176), said pin having a diameter of at least 1.5 mm., said second punch (172) further having a shoulder (182) formed at an opposite end of said tubular section (174) which acts as a stop for said tube (120); and
d) a second die (186) mateable with said second punch (172) to transform said crimped end of said tube (120) into a plurality of pleats (134) and form said pleats (134) into a semi-spherically shaped tip (190) having a central aperture (192) formed therethrough, said second die (186) including a base (194) having a semi-spherical cavity (200) formed therein with a central passageway (202) formed at the bottom of said cavity (200), said cavity (200) being sized to receive both said second punch (172) and said tube (120) and said passageway (202) being sized to receive only said pin (178).

24. The apparatus of claim 23, wherein said pin (178) has a diameter of at least 0.32 cm (0.125 inches).

25. The apparatus of claim 23, wherein said pin (178) has a circular cross-sectional configuration.

26. The apparatus according to claims 23 to 25, wherein said first punch (144) further has a configured tip (148) with a plurality of elongated grooves (150) formed therein, and wherein each of said blades (168) is capable of engaging with one of said grooves (150) formed on said first punch (144).

27. The apparatus of claim 26, wherein said configured tip (148) formed on said first punch (144) contains a knurled area (154) which facilitates crimping said tube (120) when said first punch (144) is brought in contact with said first die (158).

28. The apparatus of claim 26, wherein said semi-spherically shaped tip (176) formed on said second punch (172) contains a knurled area (184) which facilitates pleating and forming said tube (120) when said second punch (172) is brought in contact with said second die (186).

29. The apparatus of claim 26, wherein said first die (158) contains at least four blades (168).

30. The apparatus of claim 26, wherein said first die (158) contains at least eight blades (168).

31. The apparatus of claim 26, wherein said first punch (144) contains at least four elongated grooves (150).

32. The apparatus according to claims 26 to 31, wherein said punch (144) has a configured tip (148) with eight elongated grooves (150) formed therein, wherein said first die (158) includes a base (166) having eight blades (168) extending axially outward therefrom, and wherein said second die (168) is mateable with said second punch (172) to transform said crimped end of said tube (120) into eight pleats (134) and form said eight pleats (134) into a semi-spherically shaped tip (190) having a central aperture (192) formed therethrough.

33. The apparatus of claim 32, wherein said pin (178) has a diameter of at leas 0.16 cm. (0.062 inches).

34. The apparatus of claim 32, wherein said pin (178) has an essentially constant diameter.

35. The apparatus of claim 32, wherein said pin (178) has a free end and said pin tapers in diameter down from a larger diameter located adjacent to its point of attachment to said tip to a smaller diameter at its free end.

36. The apparatus of claim 32, wherein said pin (178) has a length of at least 0.15 cm (0.06 inches).

37. A method of crimping, pleating and forming a tip (190) on a hollow tube (120) using a first punch (144, 144') having a tubular section sized to receive said tube (120) and having a configured tip with a plurality of elongated grooves formed therein, and a shoulder (156) formed at an opposite end of said tubular section which acts as a stop for said tube (120), a first die (158) mateable with said first punch (144, 144') and tube (120) to crimp an end of said tube (120), said first die (158) including a base having a plurality of blades extending axially outward therefrom, each of said blades capable of engaging with one of said grooves formed on said first punch (144, 144') and crimping the tip of said tube (120) positioned therebetween, a second punch (172) having a tubular section sized to receive said tube and having a semi-spherically shaped tip with a pin (178) extending outward from the apex of said tip, said pin having a diameter of at least 1.5 mm, said second punch (172) further having a shoulder (182) formed at an opposite end of said tubular section which acts as a stop for said tube (120), and a second die (186) mateable with said second punch (172) to transform said crimped end of said tube (120) into a plurality of pleats (134) and form said pleats (134) into a semi-spherically shaped tip (190) having a central aperture (192) formed therethrough, said aperture having a diameter between 1.5 mm to 5.0 mm, said second die (186) including a base having a semi-spherical cavity formed therein with a central passageway formed at the bottom of said cavity, said cavity being sized to receive both said second punch (172) and said tube (120) and said passageway being sized to receive only said pin (178), said method comprising the steps of:
a) sliding said hollow tube (120) onto said first punch (144, 144') until an end of said tube (120) contacts said shoulder (156);
b) moving said first punch (144, 144') and tube (120) into engagement with said first die (158) to form a plurality of crimps on an opposite end of said tube (120);
c) sliding said tube (120) onto said second punch (172) until the non-crimped end of said tube (120) contacts said shoulder (182); and
d) moving said second punch (172) and tube (120) into engagement with said second die (186) and engaging said pin (178) in said passageway to transform said crimped end of said tube (120) into a plurality of pleats (134) and forming said pleats (134) into a semi-spherically shaped tip (190) having a central aperture (192) formed therethrough.

38. The method of claim 37, wherein when one end of said tube (120) contacts said shoulder, the opposite end of said tube is approximately flush with said configured tip formed on said first punch (144, 144').

39. The method of claim 37, wherein said first punch (144, 144') and said first die (158) are engaged to form at least four pleats in one end of said tube (120).

40. The method of claim 37, wherein said second punch (172) and said second die are engaged to form an aperture having a circular periphery.

41. The method of claim 37, wherein said first punch (144, 144') and said first die (158) are axially engaged such that each of said blades formed on said first die axially align with one of said corresponding groves formed on said first punch (144, 144').

42. The method of any of claims 37 to 41, wherein after step b) and before step c) the step disengaging said first punch (144, 144') from said first die (158) and removing said crimped tube from said first punch; and wherein after step d) the step disengaging said second punch from said second die and removing said pleated and formed tube (120) from said second punch (172) is performed.

## Patentansprüche

1. Tampon-Applikator (10), umfassend:
a) ein erstes Glied (20), welches in der Lage ist, einen saugfähigen Tampon (12) aufzunehmen, wobei das erste Glied (20) eine mittlere Längsachse und erste (28) und zweite Enden (30) aufweist;
b) eine Einführspitze (32), welche am ersten Ende (28) des ersten Gliedes (20) einstückig angeformt ist und sich von dort nach außen erstreckt, wobei die Einführspitze (32) eine Mehrzahl von Falten (54) aufweist, welche in der Lage sind, sich nach außen zu dehnen, wenn der Tampon (12) aus dem ersten Glied (20) ausgestoßen wird; und
c) ein zweites Glied (22), welches im zweiten Ende (30) des ersten Gliedes (20) teleskopartig angebracht ist, wobei das zweite Glied (22) ausgebildet ist, um den Tampon (12) durch die Einführspitze (32) auszustoßen, wenn dieses in das erste Glied (20) geschoben wird, wobei der Tampon-Applikator (10) dadurch gekennzeichnet ist, dass die Einführspitze (32) einen halbkugelförmigen Abschnitt (34) und einen kegelstumpfförmigen Abschnitt (36) umfasst, wobei der halbkugelförmige Abschnitt (34) eine Öffnung (40), welche dort hindurch ausgebildet ist, aufweist, wobei die Öffnung (40) eine Seitenwand (48) aufweist, welche im wesentlichen parallel zur mittleren Längsachse ausgerichtet ist, wobei die Öffnung (40) einen Durchmesser zwischen 1,5 mm und 5,0 mm aufweist, und wobei der kegelstumpfförmige Abschnitt (36) zwischen dem halbkugelförmigen Abschnitt (34) und dem ersten Ende (28) des ersten Gliedes (20) angeordnet ist.

2. Tampon-Applikator (10) nach Anspruch 1, wobei sich der kegelstumpfförmige Abschnitt (36) vom ersten Ende (28) des ersten Gliedes (20) weg nach außen erstreckt und in einem Winkel zwischen ungefähr 5° und ungefähr 40° nach innen zuläuft.

3. Tampon-Applikator (10) nach Anspruch 2, wobei der kegelstumpfförmige Abschnitt (36) in einem Winkel zwischen ungefähr 5° bis ungefähr 25° nach innen zuläuft.

4. Tampon-Applikator (10) nach Anspruch 2, wobei der kegelstumpfförmige Abschnitt (36) eine Länge von weniger als 2,54 cm (1 Inch) aufweist.

5. Tampon-Applikator (10) nach Anspruch 4, wobei sich der Durchmesser des kegelstumpfförmigen Abschnittes (36) vom Befestigungspunkt am ersten Ende (28) des ersten Gliedes (20) zum Befestigungspunkt am halbkugelförmigen Abschnitt (34) hin verkleinert.

6. Tampon-Applikator (10) nach Anspruch 1, wobei die Einführspitze (32) eine gerade Anzahl von Falten (54) aufweist.

7. Tampon-Applikator (10) nach Anspruch 1, wobei die Einführspitze (32) eine ungerade Anzahl von Falten (54) aufweist.

8. Tampon-Applikator (10) nach Anspruch 1, wobei die Einführspitze (32) drei bis zweiunddreißig Falten (54) aufweist.

9. Tampon-Applikator (10) nach Anspruch 1, wobei die Einführspitze (32) 6 bis 12 Falten (54) aufweist.

10. Tampon-Applikator (10) nach einem der vorhergehenden Ansprüche, wobei:
das erste Glied (20) eine im wesentlichen glatte äußere Oberfläche aufweist und die Einführspitze (32) mindestens drei Falten (54) umfasst, welche in der Lage sind, sich radial nach außen zu dehnen, wenn der Tampon (12) aus dem ersten Glied (20) ausgestoßen wird.

11. Tampon-Applikator (10) nach Anspruch 10, wobei jede der Falten (54) ein erstes Ende (56), welches mit der Öffnung (40) zusammenfällt, und ein zweites Ende (58), welches mit einem Punkt an einer äußeren Oberfläche des kegelstumpfförmigen Abschnittes (36) zusammenfällt, aufweist.

12. Tampon-Applikator (10) nach Anspruch 10, wobei jede der Falten (54) ein erstes Ende (56) aufweist, welches mit der Öffnung (40) zusammenfällt, und ein zweites Ende (58), welches mit einem Ort zusammenfällt, an dem der kegelstumpfförmige Abschnitt (36) mit dem ersten Glied (20) verbunden ist.

13. Tampon-Applikator (10) nach Anspruch 10, wobei jede der Falten (54) ein erstes Ende (56) aufweist, welches mit der Öffnung (40) zusammenfällt, und ein zweites Ende (58), welches mit einem Ort zusammenfällt, an dem der halbkugelförmige Abschnitt (34) mit dem kegelstumpfförmigen Abschnitt (36) verbunden ist.

14. Tampon-Applikator (10) nach Anspruch 10, wobei der kegelstumpfförmige Abschnitt (36) an einem Ende einen Außendurchmesser aufweist, welcher annähernd dem Außendurchmesser des ersten Gliedes (20) entspricht.

15. Tampon-Applikator (10) nach Anspruch 14, wobei der kegelstumpfförmige Abschnitt (36) an einem entgegengesetzten Ende einen Außendurchmesser aufweist, welcher annähernd dem Außendurchmesser des halbkugelförmigen Abschnittes (34) entspricht.

16. Tampon-Applikator (10) nach Anspruch 1 bis 6 und 8 bis 15, wobei das erste Glied (20) ein hohles, spiralförmig gewundenes erstes Glied ist, welches eine im wesentlichen glatte äußere Oberfläche aufweist, und die Einführspitze (32) acht Falten (54) umfasst, welche in einem annähernd gleichen Abstand voneinander beabstandet und in der Lage sind, sich radial nach außen zu dehnen, wenn der Tampon (12) aus dem ersten Glied (20) ausgestoßen wird.

17. Tampon-Applikator (10) nach Anspruch 16, wobei der kegelstumpfförmige Abschnitt (36) an einem Ende einen Außendurchmesser aufweist, welcher dem Außendurchmesser des ersten Gliedes (20) annähernd entspricht.

18. Tampon-Applikator (10) nach Anspruch 16, wobei das erste Glied (20) einen Außendurchmesser aufweist und der halbkugelförmige Abschnitt (34) einen Durchmesser aufweist, welcher im Bereich von ungefähr 50 % bis ungefähr 90 % des Außendurchmessers des ersten Gliedes (20) liegt.

19. Tampon-Applikator (10) nach Anspruch 16, wobei die Seitenwand (48) der Öffnung (40) einen Radius aufweist, welcher einer äußeren Oberfläche der Einführspitze (32) benachbart ausgebildet ist.

20. Tampon-Applikator (10) nach Anspruch 16, wobei die Falten (54) eine Dicke von weniger als 0,5 mm aufweisen.

21. Kombination aus einem Tampon-Applikator (110) und einem Menstruations-Tampon (112) mit einer geformten Nase, wobei die Kombination umfasst:
a) ein erstes Glied (120), welches in der Lage ist, den Menstruations-Tampon (112) aufzunehmen, wobei das erste Glied (120) eine mittlere Längsachse und erste (128) und zweite Enden (130) aufweist;
b) eine Einführspitze (132), welche am ersten Ende (128) des ersten Gliedes (120) einstückig angeformt ist und sich von dort nach außen erstreckt, wobei die Einführspitze eine Mehrzahl von Falten aufweist, welche in der Lage sind, sich nach außen zu dehnen, wenn der Tampon aus dem ersten Glied ausgestoßen wird; und
c) ein zweites Glied (122), welches im zweiten Ende (130) des ersten Gliedes (120) teleskopartig angebracht ist, wobei das zweite Glied (122) ausgebildet ist, den Tampon (112) durch die Einführspitze (132) auszustoßen, wenn dieses in das erste Glied (120) geschoben wird;
wobei die Kombination dadurch gekennzeichnet ist, dass die Einführspitze (132) des Tampon-Applikators (110) einen halbkugelförmigen Abschnitt und einen kegelstumpfförmigen Abschnitt umfasst, wobei der halbkugelförmige Abschnitt eine Öffnung (136), welche dort hindurch ausgebildet ist, aufweist, wobei die Öffnung (136) eine Seitenwand aufweist, welche im wesentlichen parallel zur mittleren Längsachse ausgerichtet ist, wobei die Öffnung einen Durchmesser zwischen 1,5 mm und 5,0 mm aufweist, und wobei der kegelstumpfförmige Abschnitt zwischen dem halbkugelförmigen Abschnitt und dem ersten Ende (128) des ersten Gliedes (120) angeordnet ist.

22. Kombination nach Anspruch 21, wobei die geformte Nase auf dem Tampon derart ausgestaltet ist, dass sie zur inneren Oberfläche der Einführspitze passt.

23. Vorrichtung zum Sicken, Fälteln und Formen einer Spitze (132) an einem hohlen Rohr (120), wobei die Vorrichtung umfasst:
a) einen ersten Stempel (144), welcher einen rohrförmigen Abschnitt (146) aufweist, welcher derart bemessen ist, dass er das Rohr (120) aufnimmt, und eine Schulter (156), welche an einem entgegengesetzten Ende des rohrförmigen Abschnittes (146) ausgebildet ist und als Anschlag für das Rohr (120) dient;
b) eine erste Matrize (158), welche mit dem ersten Stempel (144) und dem Rohr (120) zusammenpasst, um ein Ende des Rohres (120) zu sicken, wobei die erste Matrize (158) eine Grundfläche (166) umfasst, welche eine Mehrzahl von Klingen (168) aufweist, welche sich axial davon nach außen erstrecken, wobei jede der Klingen (168) in der Lage ist, mit dem Rohr (120) einzugreifen und die Spitze (132) des dazwischen angeordneten Rohres zu sicken;
c) einen zweiten Stempel (172), welcher einen rohrförmigen Abschnitt (174) aufweist, welcher derart bemessen ist, dass er das Rohr (120) aufnimmt, und eine halbkugelförmige Spitze (176) aufweist, mit einem Stift, welcher sich vom Scheitelpunkt der Spitze (176) nach außen weg erstreckt, wobei der Stift einen Durchmesser von mindestens 1,5 mm aufweist, wobei der zweite Stempel (172) des weiteren eine Schulter (182) aufweist, welche an einem entgegengesetzten Ende des rohrförmigen Abschnittes (174) ausgebildet ist und als Anschlag für das Rohr (120) dient; und
d) eine zweite Matrize (186), welche mit dem zweiten Stempel (172) zusammenpasst, um das gesickte Ende des Rohres (120) zu einer Mehrzahl von Falten (134) umzuformen und diese Falten (134) zu einer halbkugelförmigen Spitze (190) zu formen, welche eine dort hindurch ausgebildete mittige Öffnung (192) aufweist, wobei die zweite Matrize (186) eine Grundfläche (194) umfasst, welche einen darin ausgebildeten halbkugelförmigen Hohlraum (200) aufweist, mit einem mittigen Durchgang (202), welcher am Boden des Hohlraums (200) ausgebildet ist, wobei der Hohlraum (200) derart bemessen ist, dass er sowohl den zweiten Stempel (172) als auch das Rohr (120) aufnimmt, und wobei der Durchgang (202) derart bemessen ist, dass er nur den Stift (178) aufnimmt.

24. Vorrichtung nach Anspruch 23, wobei der Stift (178) einen Durchmesser von mindestens 0,32 cm (0,125 Inch) aufweist.

25. Vorrichtung nach Anspruch 23, wobei der Stift (178) eine kreisförmige Querschnitts-Konfiguration aufweist.

26. Vorrichtung nach Anspruch 23 bis 25, wobei der erste Stempel (144) des weiteren eine konfigurierte Spitze (148) mit einer darin ausgebildeten Mehrzahl länglicher Rillen (150) aufweist, und wobei jede der Klingen (168) in der Lage ist, mit einer der Rillen (150), welche auf dem ersten Stempel (144) ausgebildet sind, in Eingriff zu kommen.

27. Vorrichtung nach Anspruch 26, wobei die konfigurierte Spitze (148), welche am ersten Stempel (144) ausgebildet ist, einen gerändelten Bereich (154) umfasst, welcher das Sicken des Rohres (120) erleichtert, wenn der erste Stempel (144) mit der ersten Matrize (158) in Kontakt gebracht wird.

28. Vorrichtung nach Anspruch 26, wobei die halbkugelförmige Spitze (176), welche am zweiten Stempel (172) ausgebildet ist, einen gerändelten Bereich (184) umfasst, welcher das Fälteln und Formen des Rohres (120) erleichtert, wenn der zweite Stempel (172) mit der zweiten Matrize (186) in Kontakt gebracht wird.

29. Vorrichtung nach Anspruch 26, wobei die erste Matrize (158) mindestens vier Klingen (168) umfasst.

30. Vorrichtung nach Anspruch 26, wobei die erste Matrize (158) mindestens acht Klingen (168) umfasst.

31. Vorrichtung nach Anspruch 26, wobei der erste Stempel (144) mindestens vier längliche Rillen (150) umfasst.

32. Vorrichtung nach Anspruch 26 bis 31, wobei der Stempel (144) eine konfigurierte Spitze (148) mit acht darin ausgebildeten länglichen Rillen (150) aufweist, wobei die erste Matrize (158) eine Grundfläche (166) umfasst, welche acht Klingen (168) aufweist, welche sich axial davon nach außen erstrecken, und wobei die zweite Matrize (168) mit dem zweiten Stempel (172) zusammenpasst, um das gesickte Ende des Rohres (120) zu acht Falten (134) umzuformen und die acht Falten (134) zu einer halbkugelförmigen Spitze (190) zu formen, welche eine dort hindurch ausgebildete mittige Öffnung (192) aufweist.

33. Vorrichtung nach Anspruch 32, wobei der Stift (178) einen Durchmesser von mindestens 0,16 cm (0,062 Inch) aufweist.

34. Vorrichtung nach Anspruch 32, wobei der Stift (178) einen im wesentlichen konstanten Durchmesser aufweist.

35. Vorrichtung nach Anspruch 32, wobei der Stift (178) ein freies Ende aufweist und der Stift sich dem Durchmesser nach von einem größeren Durchmesser neben seinem Befestigungspunkt an der Spitze zu einem kleineren Durchmesser an seinem freien Ende verjüngt.

36. Vorrichtung nach Anspruch 32, wobei der Stift (178) eine Länge von mindestens 0,15 cm (0,06 Inch) aufweist.

37. Verfahren des Sickens, Fältelns und Formens einer Spitze (190) auf einem hohlen Rohr (120) unter Verwendung eines ersten Stempels (144, 144'), welcher einen rohrförmigen Abschnitt aufweist, welcher derart bemessen ist, dass er das Rohr (120) aufnimmt, und welcher eine konfigurierte Spitze mit einer Mehrzahl darin ausgebildeter länglicher Rillen aufweist, und wobei eine Schulter (156) an einem entgegengesetzten Ende des rohrförmigen Abschnittes ausgebildet ist, welche als Anschlag für das Rohr (120) dient, einer ersten Matrize (158), welche mit dem ersten Stempel (144, 144') und dem Rohr (120) zusammenpasst, um ein Ende des Rohres (120) zu sicken, wobei die erste Matrize (158) eine Grundfläche umfasst, welche eine Mehrzahl von Klingen aufweist, welche sich davon axial nach außen erstrecken, wobei jede der Klingen in der Lage ist, mit einer der Rillen, welche am ersten Stempel (144, 144') ausgebildet sind, in Eingriff zu gelangen, und die Spitze des dazwischen angeordneten Rohres (120) zu sicken, eines zweiten Stempels (172), welcher einen rohrförmigen Abschnitt aufweist, welcher bemessen ist, um das Rohr aufzunehmen, und welcher eine halbkugelförmige Spitze aufweist, mit einem Stift (178), welcher sich vom Scheitelpunkt der Spitze nach außen erstreckt, wobei der Stift einen Durchmesser von mindestens 1,5 mm aufweist, wobei der zweite Stempel (172) des weiteren eine Schulter (182) aufweist, welche an einem entgegengesetzten Ende des rohrförmigen Abschnittes ausgebildet ist, welche als Anschlag für das Rohr (120) dient, und einer zweiten Matrize (186), welche mit dem zweiten Stempel (172) zusammenpasst, um das gesickte Ende des Rohres (120) zu einer Mehrzahl von Falten (134) umzuformen und die Falten (134) zu einer halbkugelförmigen Spitze (190) zu formen, welche dort hindurch eine mittige Öffnung (192) aufweisen, wobei die Öffnung einen Durchmesser von 1,5 mm bis 5,0 mm aufweist, wobei die zweite Matrize (186) eine Grundfläche aufweist, welche einen darin ausgebildeten halbkugelförmigen Hohlraum aufweist, wobei ein mittiger Durchgang am Boden des Hohlraumes ausgebildet ist, wobei der Hohlraum bemessen ist, um sowohl den zweiten Stempel (172) als auch das Rohr (120) aufzunehmen, und der Durchgang bemessen ist, um nur den Stift (178) aufzunehmen, wobei das Verfahren die folgenden Schritte umfasst:
a) Schieben des hohlen Rohres (120) auf den ersten Stempel (144, 144'), bis ein Ende des Rohres (120) die Schulter (156) berührt;
b) Bewegen des ersten Stempels (144, 144') und des Rohres (120) in Eingriff mit der ersten Matrize (158), um eine Mehrzahl von Sicken an einem entgegengesetzten Ende des Rohres (120) auszubilden;
c) Schieben des Rohres (120) auf den zweiten Stempel (172), bis das nicht gesickte Ende des Rohres (120) die Schulter (182) berührt; und
d) Bewegen des zweiten Stempels (172) und des Rohres (120) in Eingriff mit der zweiten Matrize (186) und Ineingriffbringen des Stiftes (178) im Durchgang, um das gesickte Ende des Rohres (120) zu einer Mehrzahl von Falten (134) umzuformen, und Formen der Falten (134) zu einer halbkugelförmigen Spitze (190), welche eine dort hindurch ausgebildete mittige Öffnung (192) aufweist.

38. Verfahren nach Anspruch 37, wobei, wenn ein Ende des Rohres (120) die Schulter berührt, das entgegengesetzte Ende des Rohres mit der konfigurierten Spitze, welche am ersten Stempel (144, 144') ausgebildet ist, annähernd bündig abschließt.

39. Verfahren nach Anspruch 37, wobei der erste Stempel (144, 144') und die erste Matrize (158) miteinander in Eingriff stehen, um mindestens vier Falten in einem Ende des Rohres (120) zu bilden.

40. Verfahren nach Anspruch 37, wobei der zweite Stempel (172) und die zweite Matrize in Eingriff stehen, um eine Öffnung zu bilden, welche eine kreisförmige Peripherie aufweist.

41. Verfahren nach Anspruch 37, wobei der erste Stempel (144, 144') und die erste Matrize (158) axial in Eingriff stehen, derart, dass jede der Klingen, welche an der ersten Matrize ausgebildet sind, axial mit einer der entsprechenden Rillen ausgerichtet sind, welche am ersten Stempel (144, 144') ausgebildet sind.

42. Verfahren nach einem beliebigen der Ansprüche 37 bis 41, wobei nach Schritt b) und vor Schritt c) der Schritt, bei welchem der erste Stempel (144, 144') von der ersten Matrize (158) gelöst wird und das gesickte Rohr vom ersten Stempel entfernt wird, erfolgt; und wobei nach dem Schritt d) der Schritt, bei welchem der zweite Stempel von der zweiten Matrize gelöst wird und das gefältelte und geformte Rohr (120) von der zweiten Matrize (172) entfernt wird, durchgeführt wird.

## Revendications

1. Applicateur de tampon (10) comprenant :
a) un premier élément (20) capable d'héberger un tampon absorbant (12), ledit premier élément (20) ayant un axe longitudinal central et des première (28) et seconde (30) extrémités ;
b) une pointe d'insertion (32) formée d'un seul tenant sur ladite première extrémité (28) dudit premier élément (20) et s'étendant vers l'extérieur depuis celle-ci, ladite pointe d'insertion (32) comprenant une série de plis (54) capables de se dilater vers l'extérieur lorsque ledit tampon (12) est expulsé. dudit premier élément (20) ; et
c) un second élément (22) monté télescopiquement dans ladite seconde extrémité (30) dudit premier élément (20), ledit second élément (22) étant adapté à expulser ledit tampon (12) au travers de ladite pointe d'insertion (32) tandis qu'il est poussé dans ledit premier élément (20), ledit applicateur de tampon (10) étant caractérisé en ce que ladite pointe d'insertion (32) comprend une portion hémisphérique (34) et une portion tronconique (36), ladite portion hémisphérique (34) ayant une ouverture (40) la traversant et ladite ouverture (40) ayant une paroi latérale (48) qui est sensiblement parallèle audit axe central longitudinal, ladite ouverture (40) ayant un diamètre compris entre 1,5 mm et 5,0 mm et ladite portion tronconique (36) étant située entre ladite portion hémisphérique (34) et ladite première extrémité (28) dudit premier élément (20).

2. Applicateur de tampon (10) selon la revendication 1, dans lequel ladite portion tronconique (36) s'étend vers l'extérieur à l'écart de ladite première extrémité (28) dudit premier élément (20) et s'effile vers l'intérieur selon un angle compris entre environ 5° et environ 40°.

3. Applicateur de tampon (10) selon la revendication 2, dans lequel ladite portion tronconique (36) s'effile vers l'intérieur selon un angle compris entre environ 5° et environ 25°.

4. Applicateur de tampon (10) selon la revendication 2, dans lequel ladite portion tronconique (36) a une longueur inférieure à 2,54 cm (1 pouce).

5. Applicateur de tampon (10) selon la revendication 4, dans lequel le diamètre de ladite portion tronconique (36) va en se réduisant depuis le point de réunion à ladite première extrémité (28) dudit premier élément (20) au point de réunion à ladite portion hémisphérique (34).

6. Applicateur de tampon (10) selon la revendication 1, dans lequel ladite pointe d'insertion (32) comporte un nombre pair de plis (54).

7. Applicateur de tampon (10) selon la revendication 1, dans lequel la pointe d'insertion (32) comporte un nombre impair de plis (54).

8. Applicateur de tampon (10) selon la revendication 1, dans lequel ladite pointe d'insertion (32) comporte de 3 à 32 plis (54).

9. Applicateur de tampon (10) selon la revendication 1, dans lequel ladite pointe d'insertion (32) comporte de 6 à 12 plis (54).

10. Applicateur de tampon (10) selon l'une quelconque des revendications précédentes, dans lequel ledit premier élément (20) a une surface extérieure sensiblement lisse, et ladite pointe d'insertion (32) comporte au moins trois plis (54) capables de se dilater radialement vers l'extérieur tandis que ledit tampon (12) est expulsé dudit premier élément (20).

11. Applicateur de tampon (10) selon la revendication 10, dans lequel chacun desdits plis (54) a une première extrémité (56) qui coïncide avec ladite ouverture (40) et une seconde extrémité (58) qui coïncide avec un point sur une surface extérieure de ladite portion tronconique (36).

12. Applicateur de tampon (10) selon la revendication 10, dans lequel chacun desdits plis (54) a une première extrémité (56) qui coïncide avec ladite ouverture (40) et une seconde extrémité (58) qui coïncide avec un emplacement où ladite portion tronconique (36) est réunie audit premier élément (20).

13. Applicateur de tampon (10) selon la revendication 10, dans lequel chacun desdits plis (54) a une première extrémité (56) qui coïncide avec ladite ouverture (40) et une seconde extrémité (58) qui coïncide avec un emplacement où ladite portion hémisphérique (34) est réunie à ladite portion tronconique (36).

14. Applicateur de tampon (10) selon la revendication 10, dans lequel ladite portion tronconique (36) a un diamètre extérieur à une extrémité qui est approximativement égal au diamètre extérieur dudit premier élément (20).

15. Applicateur de tampon (10) selon la revendication 14, dans lequel ladite portion tronconique (36) a un diamètre extérieur à une extrémité opposée qui est approximativement égal au diamètre extérieur de ladite portion hémisphérique (34).

16. Applicateur de tampon (10) selon les revendications 1 à 6 et 8 à 15, dans lequel ledit premier élément (20) est un premier élément creux enroulé en spirale ayant une surface extérieure sensiblement lisse et ladite pointe d'insertion (32) comporte huit plis (54) qui sont approximativement équidistants et qui sont capables de se dilater radialement vers l'extérieur lorsque ledit tampon (12) est expulsé dudit premier élément (20).

17. Applicateur de tampon (10) selon la revendication 16, dans lequel ladite portion tronconique (36) a un diamètre extérieur à une extrémité qui est approximativement égal au diamètre extérieur dudit premier élément (20).

18. Applicateur de tampon (10) selon la revendication 16, dans lequel ledit premier élément (20) a un diamètre extérieur et ladite portion hémisphérique (34) a un diamètre qui est compris entre environ 50% et environ 90% dudit diamètre extérieur dudit premier élément (20).

19. Applicateur de tampon (10) selon la revendication 16, dans lequel ladite paroi latérale (48) de ladite ouverture (36) contient un rayon formé au voisinage d'une surface extérieure de ladite pointe d'insertion (32).

20. Applicateur de tampon (10) selon la revendication 16, dans lequel lesdits plis (54) ont une épaisseur inférieure à 0,5 mm.

21. A titre de combinaison, un applicateur de tampon (110) et un tampon cataménial (112) ayant un nez conformé, ladite combinaison comprenant :
a) un premier élément (120) capable d'héberger ledit tampon cataménial (112), ledit premier élément (120) ayant un axe central longitudinal et des première (128) et seconde (130) extrémités ;
b) une pointe d'insertion (132) formée d'un seul tenant sur ladite première extrémité (128) dudit premier élément (120) et s'étendant à l'extérieur de celle-ci, ladite pointe d'insertion comportant une série de plis qui sont capables de se dilater radialement vers l'extérieur lorsque ledit tampon est expulsé dudit premier élément ; et
c) un second élément (122) monté télescopiquement dans ladite seconde extrémité (130) dudit premier élément (120), ledit second élément (122) étant adapté à expulser ledit tampon (112) au travers de ladite pointe d'insertion (132) tandis qu'il est poussé dans ledit premier élément (120) ;
ladite combinaison étant caractérisée en ce que ladite pointe d'insertion (132) dudit applicateur de tampon (110) comprend une portion hémisphérique et une portion tronconique, ladite portion hémisphérique ayant une ouverture (136) formée à travers elle et ladite ouverture (136) ayant une paroi latérale qui s'étend essentiellement parallèlement audit axe central longitudinal, ladite ouverture ayant un diamètre compris entre 1,5 mm et 5,0 mm, et ladite portion tronconique étant située entre ladite portion hémisphérique et ladite première extrémité (128) dudit premier élément (120).

22. Combinaison selon la revendication 21, dans laquelle ledit nez conformé sur ledit tampon est configuré pour se conformer à ladite surface intérieure de ladite pointe d'insertion.

23. Appareil pour sertir, plisser et former une pointe (132) sur un tube creux (120), ledit appareil comprenant :
a) un premier poinçon (144) ayant une section tubulaire (146) dimensionnée pour recevoir ledit tube (120) et un épaulement (156) formé au niveau d'une extrémité opposée de ladite section tubulaire (146) qui agit comme butée pour ledit tube (120) ;
b) une première matrice (158) susceptible d'être appariée avec ledit premier poinçon (144) et ledit tube (120) pour sertir une extrémité dudit tube (120), ladite première matrice (158) comprenant une base (166) ayant une série de lames (168) s'étendant axialement vers l'extérieur depuis celle-ci, chacune desdites lames (168) étant capable de venir en prise avec ledit tube (120) et de sertir la pointe (132) dudit tube positionné entre elles ;
c) un second poinçon (172) ayant une section tubulaire (174) dimensionné pour recevoir ledit tube (120) et ayant une pointe de forme hémisphérique (176) avec une tige s'étendant vers l'extérieur depuis le sommet de ladite pointe (176), ladite tige ayant un diamètre d'au moins 1,5 mm, ledit second poinçon (172) comprenant en outre un épaulement (182) formé au niveau d'une extrémité opposée de ladite section tubulaire (174) qui agit comme butée pour ledit tube (120) ; et
d) une seconde matrice (186) susceptible d'être appariée avec ledit second poinçon (172) pour transformer ladite extrémité sertie dudit tube (120) en une série de plis (134) et pour transformer lesdits plis (134) en une pointe de forme hémisphérique (190) ayant une ouverture centrale (192) formée à travers elle, ladite seconde matrice (186) comprenant une base (194) ayant une cavité hémisphérique (200) formée en son sein avec un passage central (202) formé au fond de ladite cavité (200), ladite cavité (200) étant dimensionnée pour recevoir à la fois ledit second poinçon (172) et ledit tube (120), et ledit passage (202) étant dimensionné pour ne recevoir que la tige (178).

24. Appareil selon la revendication 23, dans lequel ladite tige (178) a un diamètre d'au moins 0,32 cm (0,125 pouce).

25. Appareil selon la revendication 23, dans laquelle ladite tige (178) a une section transversale circulaire.

26. Appareil selon les revendications 23 à 25, dans lequel ledit premier poinçon (144) comprend en outre une pointe configurée (148) avec une série de rainures allongées (150) formées dans ladite pointe, et dans lequel chacune desdites lames (168) est capable de venir en prise avec l'une desdites rainures (150) formées sur ledit premier poinçon (144).

27. Appareil selon la revendication 26, dans lequel ladite pointe configurée (148) formée sur ledit premier poinçon (144) présente une zone moletée (154) qui facilite le sertissage dudit tube (120) lorsque ledit premier poinçon (144) est amené en contact avec ladite première matrice (158).

28. Appareil selon la revendication 26, dans lequel ladite pointe de forme hémisphérique (176) formée sur ledit second poinçon (172) présente une zone moletée (184) qui facilite le plissage et la formation dudit tube (120) lorsque ledit second poinçon (172) est amené en contact avec ladite seconde matrice (186).

29. Appareil selon la revendication 26, dans lequel ladite première matrice (158) comporte au moins quatre lames (168).

30. Appareil selon la revendication 26, dans lequel ladite première matrice (158) comporte au moins huit lames (168).

31. Appareil selon la revendication 26, dans lequel ledit premier poinçon (144) comporte au moins quatre rainures allongées (150).

32. Appareil selon les revendications 26 à 31, dans lequel ledit poinçon (144) a une pointe configurée (148) avec huit rainures allongées (150) formées dans ladite pointe, ladite première matrice (158) comprenant une base (166) ayant huit lames (168) s'étendant axialement vers l'extérieur depuis celle-ci, et dans lequel ladite seconde matrice (186) est susceptible d'être appariée avec ledit second poinçon (172) pour transformer ladite extrémité sertie dudit tube (120) en huit plis (134) et pour transformer lesdits huit plis (134) en une pointe de forme hémisphérique (190) ayant une ouverture centrale (192) formée à travers ladite pointe.

33. Appareil selon la revendication 32, dans lequel ladite tige (178) a un diamètre d'au moins 0,16 cm (0,062 pouce).

34. Appareil selon la revendication 32, dans lequel ladite tige (178) a un diamètre essentiellement constant.

35. Appareil selon la revendication 32, dans lequel ladite tige (178) a une extrémité libre et dans lequel le diamètre de ladite tige va en s'amincissant depuis un plus grand diamètre situé au voisinage de son point de réunion à ladite pointe vers un plus petit diamètre à son extrémité libre.

36. Appareil selon la revendication 32, dans lequel ladite tige (178) a une longueur d'au moins 0,15 cm (0,06 pouce).

37. Procédé de sertissage, plissage et formation d'une pointe (190) sur un tube creux (120) utilisant un premier poinçon (144, 144') ayant une section tubulaire dimensionnée pour recevoir ledit tube (120) et ayant une pointe configurée avec une série de rainures allongées formées dans ladite pointe, et un épaulement (156) formé au niveau d'une extrémité opposée de ladite section tubulaire qui agit comme butée pour ledit tube (120), une première matrice (158) susceptible d'être appariée avec ledit premier poinçon (144, 144') et ledit tube (120) pour sertir une extrémité dudit tube (120), ladite première matrice (158) comprenant une base ayant une série de lames s'étendant axialement vers l'extérieur depuis ladite base, chacune desdites lames étant capable de venir en prise avec l'une desdites rainures formées sur ledit premier poinçon (144, 144') et de sertir la pointe dudit tube (120) positionné entre elles, un second poinçon (172) ayant une section tubulaire dimensionnée pour recevoir ledit tube et ayant une pointe de forme hémisphérique avec une tige (178) s'étendant vers l'extérieur depuis le sommet de ladite pointe, ladite tige ayant un diamètre d'au moins 1,5 mm, ledit second poinçon (172) comprenant en outre un épaulement (182) formé au niveau d'une extrémité opposée de ladite section tubulaire qui agit comme butée pour ledit tube (120), et une seconde matrice (186) susceptible d'être appariée avec ledit second poinçon (172) pour transformer ladite extrémité sertie dudit tube (120) en une série de plis (134) et transformer lesdits plis (134) en une pointe de forme hémisphérique (190) ayant une ouverture centrale (192) formée au travers de ladite pointe, ladite ouverture ayant un diamètre compris entre 1,5 mm et 5,0 mm, ladite seconde matrice (186) comprenant une base ayant une cavité hémisphérique formée en son sein avec un passage central formé au fond de ladite cavité, ladite cavité étant dimensionnée pour recevoir à la fois ledit second poinçon (172) et ledit tube (120), et ledit passage étant dimensionné pour ne recevoir que ladite tige (178), ledit procédé comprenant les étapes suivantes :
a) l'enfilage dudit tube creux (120) sur ledit premier poinçon (144, 144') jusqu'à ce qu'une extrémité dudit tube (120) vienne en contact avec ledit épaulement (156) ;
b) le déplacement dudit premier poinçon (144, 144') et dudit tube (120) pour les amener en contact avec ladite première matrice (158) pour former une série de plis de sertissage sur une extrémité opposée dudit tube (120) ;
c) l'enfilage dudit tube (120) sur ledit second poinçon (172) jusqu'à ce que l'extrémité non sertie dudit tube (120) vienne contact avec ledit épaulement (182) ; et
d) l'amenée dudit second poinçon (172) et dudit tube (120) en prise avec ladite seconde matrice (186) et la venue en prise de ladite tige (178) avec ledit passage pour transformer ladite extrémité sertie dudit tube (120) en une série de plis (134) et la transformation desdits plis (134) en une pointe de forme hémisphérique (190) ayant une ouverture centrale (192) formée à travers ladite pointe.

38. Procédé selon la revendication 37, dans lequel, lorsque l'une des extrémités dudit tube (120) vient en contact avec ledit épaulement, l'extrémité opposée dudit tube affleure approximativement ladite pointe configurée formée sur ledit premier poinçon (144, 144').

39. Procédé selon la revendication 37, dans lequel ledit premier poinçon (144, 144') et ladite première matrice (158) viennent en prise pour former au moins quatre plis dans une extrémité dudit tube (120).

40. Procédé selon la revendication 37, dans lequel ledit second poinçon (172) et ladite seconde matrice sont amenés en prise pour former une ouverture ayant une périphérie circulaire.

41. Procédé selon la revendication 37, dans lequel ledit premier poinçon (144, 144') et ladite première matrice (158) viennent en prise axiale de telle sorte que chacune desdites lames formées sur ladite première matrice est alignée axialement avec l'une desdites rainures correspondantes formées sur ledit premier poinçon (144, 144').

42. Procédé selon l'une quelconque des revendications 37 à 41, qui comporte, après l'étape b) et avant l'étape c), l'étape de désengagement dudit premier poinçon (144, 144') d'avec ladite première matrice (158) et la séparation dudit tube serti d'avec ledit premier poinçon ; et, après l'étape b), l'étape de désengagement dudit second poinçon d'avec ladite seconde matrice et la séparation dudit tube plissé et conformé (120) d'avec ledit second poinçon (172).
